# EUROPEAN PATENT APPLICATION

(11) **EP 3 872 088 A1**
(43) Date of publication of application: **01.09.2021**
(21) Application number: 20305209.7
(22) Date of filing: 28.02.2020
(51) Int. Cl.: C07K 14/81, C12N 15/82, C12N 1/12

(54) **USE OF APROTININ AS A CARRIER TO PRODUCE A RECOMBINANT PROTEIN, POLYPEPTIDE OR PEPTIDE IN ALGAE**

(71) Applicant: Alganelle, 73370 Bourget Du Lac (FR)
(72) Inventor: TISSOT-LECUELLE, Ghislaine, 73290 La Motte Servolex (FR)
(74) Representative: Lavoix

(57) **Abstract**

The present invention relates to the use of aprotinin as a carrier to produce a recombinant protein, polypeptide or peptide in algae, in particular microalgae, wherein aprotinin and said recombinant protein, polypeptide or peptide are fused together to form a fusion protein. It also relates to a method to produce a recombinant protein, polypeptide or peptide in algae, wherein said method comprises genetic transformation of algae, in particular microalgae, with a recombinant nucleic acid sequence encoding a fusion protein, wherein said fusion protein comprises aprotinin and said recombinant protein, polypeptide or peptide. It further relates to a recombinant algae comprising a recombinant nucleic acid sequence encoding a fusion protein, wherein said fusion protein comprises aprotinin and a recombinant protein, polypeptide or peptide. The use of said recombinant algae, for producing said fusion protein is also contemplated.

## Description

The present invention relates to the use of aprotinin as a carrier to produce a recombinant protein, polypeptide or peptide in algae, in particular microalgae, wherein aprotinin and said recombinant protein, polypeptide or peptide are fused together to form a fusion protein. It also relates to a method to produce a recombinant protein, polypeptide or peptide in algae, wherein said method comprises genetic transformation of algae, in particular microalgae, said algae comprising a recombinant nucleic acid sequence encoding a fusion protein, wherein said fusion protein comprises aprotinin and said recombinant protein, polypeptide or peptide. It further relates to a recombinant algae comprising a recombinant nucleic acid sequence encoding a fusion protein, wherein said fusion protein comprises aprotinin and a recombinant protein, polypeptide or peptide. The use of said recombinant algae, for producing said fusion protein is also contemplated.

Aprotinin, also known as basic pancreatic trypsin inhibitor (BPTI), is a small single-chain polypeptide cross-linked by three disulfide bridges. It consists of 58 amino acid residues with a molecular mass of 6.5 kDa and an isoelectric point of 10.9.

Aprotinin is a Kunitz-type inhibitor with a relatively broad specificity, which inhibits a range of serine proteases including trypsin, chymotrypsin, plasmin, kallikrein, enterokinase and thrombin.

Accordingly, it can be used as an affinity ligand for the purification of these proteases. As a potent proteolytic inhibitor, aprotinin is widely used to prevent degradation of target polypeptides in protein preparation.

Clinically, aprotinin is used to prevent excessive loss of blood in cardiac surgery and organ transplantation.

In recent years, the demand for recombinant protein is increasing more and more because of their high value applications in broad range industries as in personal care, cosmetics, healthcare, tissue engineering, biomaterials, agriculture and paper industries. Numerous examples of commercial pharmaceutical proteins produced in various recombinant systems have been launched, as for instance, insulin, human growth hormone, erythropoietin and interferon.

Additionally, large quantities of proteins and peptides are needed for these various industrial applications.

The most current industrial expression systems include the bacteria *E. coli,* the yeast (*S. cerevisiae* and *P. pastoris*) and mammalian cell lines. Emerging technologies are insect cell cultures, plants and microalgae.

However, the expression of recombinant peptides and proteins is still limited, as large efforts are required in order to obtain the desired peptides and proteins with a native conformation, in high amounts and high purity. Even a current bacterial system such as *E. coli* has limitations at expressing recombinant peptides/polypeptides/proteins. Indeed, formation of insoluble aggregates (or inclusion body) arises due to the lack of sophisticated machinery to perform posttranslational modifications, as for instance disulfide bound formation or glycosylations. This results in poor solubility of the protein of interest and/or in the absence of protein activity.

Interest in microalgae as an alternative platform for recombinant protein production has been gaining in recent years.

Recombinant algae offer several advantages over the other recombinant protein production platforms. Microalgae are photosynthetic unicellular microorganisms, with low nutriment requirements to grow. They are capable of photoautotrophic, mixotrophic or heterotrophic growth. The cost of protein production in algae is much lower than other production systems in photoauxotrophic growth. Proteins purified from algae, as from plant, should be free from toxins and viral agents that may be present in preparations from bacteria or mammalian cell culture. Indeed, several microalgae species have the GRAS status (Generally Recognized As Safe) Granted by the FDA, as for instance for microalgae, *Chlorella vulgaris, Chlorella protothecoides* S106, *Dunaliella bardawil, Chlamydomonas reinhardtii* and for cyanobacteria *Arthrospira plantesis.*

As in transgenic plants, algae have been engineered, to express recombinant genes from both the nuclear and chloroplast genomes.

In addition, recombinant synthesis of peptides and polypeptides composed of repeating units of specific amino acid sequence is difficult to express, because the peptide encoding DNA sequences are often subject to genetic recombination resulting in genetic instability and leading often to the production of proteins smaller than the native one.

Recombinant production of relatively small peptides can be also challengeous because they can self-assemble or be subject to proteolytic degradation.

Moreover, in contrast to plant expression system, algae are robust industrial chassis with competitive production costs reachable at industrial scale in reproducible, sterile and well-controlled production conditions within photobioreactors and fermenters or in a single use wave bag. In addition, they can secrete recombinant proteins outside the cell and thus in the culture media, simplifying the subsequent purification steps. Algae have no seasonality and don't used arable land.

The development of chloroplast transformation in algae for the production of proteins of interest is more recent than in plants and requires improvement. In fact, the production yield of recombinant protein are typically between 0.5 and 5% of the total soluble proteins in *Chlamydomonas reinhardtii* chloroplast, which is still low in comparison to established platform.

In addition, some mammalian proteins are not easily expressed in *Chlamydomonas reinhardtii* (Rasala *et al.,* 2010).

Fusion partners or carriers have been developed in recombinant protein production in order to increase accumulation yields, and/or solubility and/or folding and/or to facilitate protein purification. Fusion partners of different sizes (or molecular weight) have been used in various production systems in order to enhance protein solubility and accumulation (maltose-binding protein (MBP), glutathione-S-transferase (GST), thioredoxin, GB1, N-utilizing substance A (NusA), ubiquitin, small ubiquitin-like modifier (SUMO), Fh8) and to facilitate detection and purification (as for examples without limitation MBP, GST and small epitope Tag peptides as c-myc Tag, poly-histidine Tag (His Tag), Flag Tag, HA Tag. Another type of fusion Tags used for purification are stimulus response Tags (or environmentally responsive polypeptides) which allow precipitation of the fusion protein when stimulus as modification of temperature or solution ionic strength are adjusted.

The inventors of the present invention have surprisingly found that aprotinin can be used as a carrier to produce a recombinant protein, polypeptide or peptide in algae, wherein aprotinin and said recombinant protein, polypeptide or peptide are fused together to form a fusion protein.

The present invention thus relates to the use of aprotinin as a carrier to produce a recombinant protein, polypeptide or peptide in algae, wherein aprotinin and said recombinant protein, polypeptide or peptide are fused together to form a fusion protein.

In particular, according to the invention, aprotinin is used as a fusion partner to improve the accumulation and/or the stability of recombinant peptides, polypeptides and proteins.

If further relates to a method to produce a recombinant protein, polypeptide or peptide in algae, wherein said method comprises genetic transformation of algae comprising a nucleic acid sequence encoding a fusion protein, wherein said fusion protein comprises aprotinin and said recombinant protein, polypeptide or peptide.

It also relates to a recombinant algae comprising a nucleic acids sequence encoding a fusion protein, wherein said fusion protein comprises aprotinin and a recombinant protein, polypeptide or peptide.

It further relates to the use of said recombinant algae, for producing said fusion protein.

As already mentioned above by "aprotinin" is meant the basic pancreatic trypsin inhibitor (BPTI), a small single-chain protein cross-linked by three disulfide bridges which consists of 58 amino acid residues with a molecular mass of 6.5 kDa and an isoelectric point of 10.9.

Said protein is well known by the man skilled in the art and is available commercially. It can for example be produced in recombinant systems such as plants (in cytoplasm by nuclear transformation (Pogue *et al.,* 2010), or in thylakoid lumens by chloroplast transformation (Tissot *et al.,* 2008).

Its formula is C₂₈₄H₄₃₂N₈₄O₇₉S₇ and its molar mass 6511.51 g/mol g·mol⁻¹.

The amino acid sequence for aprotinin from *Bos Taurus* (bovine) is RPDFC LEPPY TGPCK ARIIR YFYNA KAGLC QTFVY GGCRA KRNNF KSAED CMRTC GGA (SEQ ID N°1; UniProt P00974; nucleic acid sequence coding for aprotinin is SEQ ID N°2).

In the context of the present invention, the term "aprotinin" also covers chimeric aprotinin and mutated aprotinin.

By "chimeric aprotinin" is meant that aprotinin is connected at its N-terminus and/or at its C-terminus to an epitope Tag peptide(s) and/or signal peptide and/or protease recognition cleavage site.

By "mutated" aprotinin is meant that the nucleic or amino acid sequence of aprotinin or chimeric aprotinin comprises one or more mutations or one amino acid/nucleic acid or more. These mutations include deletions, substitutions, insertions and /or cleavage of one or more nucleic acids or amino acids. The supplementary amino acids/nucleic acids can be any amino acids/nucleic acids.

The chimeric aprotinin can be for example the protein called HA-APRO (SEQ ID N°3) comprising aprotinin fused at its N-terminus to the HA epitope Tag or 3F-APRO (SEQ ID N°4) comprising aprotinin fused at its N-terminus to the 3XFlag epitope Tag (3F).

Other examples of chimeric aprotinin can be the protein called HA-SP-3F-FX-APRO (SEQ ID N°5 and 6) comprising aprotinin fused at its N-terminus to an amino acid sequence containing the HA Tag (HA) peptide followed by the signal peptide (SP), a 3XFlag Tag (3F) and a cleavage site for Factor Xa protease (FX); the chimeric aprotinin called HA-SP-3F-APRO (SEQ ID N° 7 and 8) comprising aprotinin fused at its N-terminus with the HA Tag followed by the signal peptide SP and the 3XFlag Tag (3F); a chimeric aprotinin called HA-SP-APRO (SEQ ID N° 9 and 10) comprising aprotinin fused at its N-terminus with a HA Tag followed by the signal peptide SP.

By "signal peptide" (SP) is meant, in the context of the present invention, an amino acid sequence placed at the N-terminus of a newly synthetized recombinant protein. This signal peptide should allow the translocation and the accumulation of the protein inside the lumen of chloroplast thylakoids and not in the chloroplast stroma. The signal peptide is cleaved after translocation across the thylakoid membranes. Two different prokaryotic-systems have been characterized for translocation of soluble proteins to the thylakoid lumen of chloroplast. The major one is the twin-arginine protein translocation (Tat) pathway. This thylakoid transport system exhibits two unusual characteristics. Firstly, it accepts proteins with an N-terminal signal peptide that carries the canonical twin-arginine motif. Second, the Tat system is capable of transporting fully folded proteins. In contrast, the second thylakoid transport system, the Sec pathway translocates proteins in their unfolded state.

For instance, signal peptides can derive from algae proteins localized in the thylakoid lumen, as the signal peptide from the *Chlamydomonas reinhardtii* 16 and 23 kDa subunits of the oxygen-evolving complex of photosystem II, or the *Chlamydomonas reinhardtii* Rieske subunit of b₆f complex or the cryptophytes phycoerythrin alpha subunit (as example from *Guillardia theta*).

In one embodiment of the present invention, the nucleic acid sequence (SEQ ID N°11) encoding the Signal Peptide (SP; SEQ ID N°12: NNNDLFQASRRRFLAQLGGLTVAGMLGPSLLTPRRATAAQA) is extracted from the sequence of the *E. coli TorA* gene encoding the Trimethylamine-N-oxide reductase 1 (UniProt number P33225). This signal peptide used the Tat sytem. This amino acid sequence is cleaved from the protein after the translocation of the later one across the thylakoid membrane. The cleavage site at the C-terminus of the SP is ATAAQA (SEQ ID N°13) (Buchanan et al., 2008; FEBS Letters 582:3979-3984).

Other signal peptides can be used which don't leave supplementary amino acids at the N-terminus of the recombinant protein as in particular signal peptide from algae, and in particular from *Chlamydomonas reinhardtii.*

If the signal peptide is cleaved after translocation into the lumen of thyalkoïds (or across the thylakoid membranes), two other chimeric aprotinins can be produced *in vivo* 3F-APRO (SEQ ID N°4) or 3F-FX-APRO (SEQ ID N°14).

In the context of the invention, by "carrier" is meant in particular that aprotinin is a fusion partner that will allow the production of a recombinant peptide, polypeptide or protein according to the invention in algae, in particular in microalgae.

In particular, aprotinin allows to improve the production of a recombinant peptide, polypeptide or protein according to the invention in algae, in particular in microalgae.

More particularly, aprotinin is used to increase accumulation and/or stability and/or solubility and/or folding and/or activity of recombinant proteins peptide, polypeptide or protein according to the invention in algae, in particular in microalgae.

According to the invention, aprotinin thus allows the production of a recombinant protein, polypeptide or peptide in algae, in particular, to overproduce said recombinant protein, polypeptide or peptide in algae.

By "overproduce" or "increased accumulation" or "accumulation increase" is notably meant that the production of said recombinant protein, polypeptide or peptide is enhanced and that said recombinant protein, polypeptide or peptide is accumulated in the algae.

It means in particular that said recombinant protein, polypeptide or peptide is produced in higher amounts than without aprotinin.

The invention thus also relates to the use according to the invention or the method according to the invention, to overproduce said recombinant protein, polypeptide or peptide in algae.

In particular, the use of aprotinin allows to improve the accumulation and/or the stability of the recombinant peptide, polypeptide or protein according to the invention in algae, in particular in microalgae.

In the context of the present invention, the production takes place in algae, in particular in microalgae.

Examples of algae that are in the scope of the invention are chosen from the group consisting of *Chlorophyta* (green algae), *Rhodophyta* (red algae), *Stramenopiles* (heterokonts), *Xanthophyceae* (yellow-green algae), *Glaucocystophyceae* (glaucocystophytes), *Chlorarachniophyceae* (chlorarachniophytes), *Euglenida* (euglenids), *Haptophyceae* (coccolithophorids), *Chrysophyceae* (golden algae), *Cryptophyta* (cryptomonads), *Dinophyceae* (dinoflagellates), *Haptophyceae* (coccolithophorids), *Bacillariophyta* (diatoms), *Eustigmatophyceae* (eustigmatophytes), *Raphidophyceae* (raphidophytes), *Scenedesmaceae, Phaeophyceae* (brown algae) and *cyanophyceae.*

In particular, those algae are microalgae.

"Microalgae" is an eukarytotic microbial organism that contains a chloroplast or plastid, and optionally that is capable of performing photosynthesis, or a prokaryotic microbial organism (cyanobacteria) capable of performing photosynthesis.

More particularly, said microalgae is chosen from the group consisting of *Chlamydomonas, Chlorella, Dunaliella, Haematococcus,* diatoms, *Scenedesmaceae, Tetraselmis, Ostreococcus, Porphyridium, Nannochloropsis, Arthrospira platensis, Arthrospira maxima, Anabaena* sp. PCC7120, Leptolyngbya sp, *Synechocystis sp,* and *Synechococcus sp.*

Even more particularly said microalgae is chosen from the group consisting of *Chlamydomonas,* more particularly *Chlamydomonas reinhardtii,* even more particularly *Chlamydomonas reinhardtii 137c or a deficient strain as Chlamydomonas reinhardtii CW15.*

In particular, said recombinant protein, polypeptide or peptide is produced or overproduced in the chloroplast of microalgae.

By "peptides", "polypeptides", "proteins" is meant the meaning commonly understood by a person skilled in the art to which this invention belongs. In particular, peptides, polypeptides and proteins are amino acid polymers linked *via* peptide (amide) bonds.

More particularly, proteins according to the invention have unique and stable three-dimensional structure and are composed of more than 50 amino acids, like proteins of 54, 60, 66, 72, 75, 78, 84, 90, 96, 100, 102, 108, 114, 120, 150, 180, 200, 300, 350 or more amino acids; peptides according to the invention are of short oligopeptides, for examples peptides of 2 to 10 amino acids, like peptides of 4, 5, 6, 7, 8, 9 or 10 amino acids; polypeptides according to classical meaning can be composed of 11 to 50 amino acids, like polypeptides of 11, 12, 15, 18, 20, 24, 25, 30, 35, 36, 40, 42, 45, 48, or 50 amino acids.

In the context of the invention, polypeptides and proteins according to the invention can contain a repetition of n units of identical or different amino acids sequence, or a repetition of n units of identical or different peptide, n being from 2 to 400.

The peptides or polypeptides according to the invention can be used in compositions. Said compositions can contain other different peptide(s) allowing the design of different biological and physical properties. In that case, peptides or polypeptides can be designed to comprise silk peptides, or the domain for the fixation of biotin or hyaluronic acid, or heparin binding domains, or growth factors, or protease degradation sites or cell binding domains (as for instance the RGD domain involved in the reconnaissance of fibrillins.

In particular, proteins according to the invention are elastin, elastin like proteins, collagen, or collagen like proteins.

In particular, polypeptides according to the invention are collagen, collagen like polypeptides or matricins polypeptides such as elastin polypeptides or elastin like polypeptides.

In particular, peptides according to the invention are collagen, collagen like peptides or matricins peptides such as elastin peptides or elastin like peptides.

In one embodiment, the peptides/polypeptides/proteins according to the invention, can be chemically modified at their N- or C-terminus by adding for instance a palmitoyl group or a hydroxyl group or an alkoyl chain, or a biotinyl group.

Matricins peptides and polypeptides are signal peptides (Schagen *et al.,* 2017). They are released from the extracellular matrix for instance during the aging process or after exposure to UVB-irradiation. Signal peptides creates a response in the damage skin dermis that induce an increase of collagen, elastin, proteoglycan and glycosaminoglycan.

In particular, matricins can be for instance Palmitoyl pentapeptide-4, (Matrixyl®) SEQ ID N°15: Pal-Lys-Thr-Thr-Lys-Ser-OH or pal-KTTKS-OH; Katayama, *et al.,* 1993; Lindner US 6,620,419 patent), Palmitoyl Hexapeptide-15 (SEQ ID N°16: Pal-GKTTKS) or Palmitoyl hexapeptide-12 (Biopeptide-EL® (SEQ ID N°17: Pal-Val-Gly-Val-Ala-Pro-Gly).

More particularly, in the present invention, peptides can be the matricins peptides KTTKS (named NY2 of SEQ ID N°18) or the peptide GKTTKS (GNY2 of SEQ ID N°19) or their derivatives.

Still more particularly, polypeptides of the invention can be polypeptides of KTTKS or of GKTTKS, or their derivatives. Such polypeptides consist in repeated units of SEQ ID N°18 or 19, i.e. they contain several fold n repeats of the peptide KTTKS or GKTTKS, or of their derivatives.

By "derivatives" of peptide or polypeptide is meant that the amino acid sequence of the native peptide or polypeptides is mutated or contains one supplementary amino acid or more at its N- or C-terminus. This supplementary amino-acids can be any amino acids.Said mutations include deletions, substitutions, insertions and /or cleavage of one or more nucleic acids or amino acids.

In particular, derivatives of KTTKS contain the KTTKS sequence with one or more amino acids at its N- and/or C-terminus (they are called NY3).

In particular, the supplementary amino-acids in the derivatives of KTTKS can be an aspartic acid or a glutamic acid.

In particular, in the present invention, the derivatives of KTTKS can be KTTKSD (named NY3a of SEQ ID N°20), KTTKSE (NY3b of SEQ ID N°21), DKTTKS (of SEQ ID N°22) or EKTTKS (of SEQ ID N°23).

In the present invention, the derivatives of GKTTKS can be GKTTKSD (of SEQ ID N°24) or GKTTKSE (of SEQ ID N°25).

As previously mentioned, polypeptides of KTTKS named (KTTKS)n contain several fold (n) repeats of the peptide KTTKS.

Polypeptides of KTTKS derivatives named (NY3a)n and (NY3b)n contain several fold (n) repeats of the peptide NY3a or NY3b.

In particular, in the present invention, polypeptides of KTTKS derivatives can contained a repetition of 5 units of NY3a or NY3b and are named respectively ((NY3a)x5 and (NY3b)x5).

More particularly particular, polypeptides derivatives according to the invention are KTTKSDKTTKSDKTTKSDKTTKSDKTTKSD ((NY3a)x5 of SEQ ID N°26) or GKTTKSDKTTKSDKTTKSDKTTKSDKTTKSD ((GNY3a)x5 of SEQ ID N°27) or KTTKSEKTTKSEKTTKSEKTTKSEKTTKSE ((NY3b)x5 of SEQ ID N°28) or GKTTKSEKTTKSEKTTKSEKTTKSEKTTKSE ((GNY3b)x5 of SEQ ID N°29).

Elastin is a major structural protein of the extracellular matrix. It is present in the connective tissue of all vertebrates providing elasticity to tissues. Elastin is firstly synthetized as soluble monomer precursors, tropoelastin, which is subsequently assembled into the mature elastin, a stable polymeric structure.

This protein is well known in the art. The amino acid sequence of elastin and tropoelastin contains short repetitive amino acid motifs and numerous hydrophobic residues. During the aging process or after exposure to UVB-irradiation or in pathological processes, elastin is degraded into short peptides, named "elastin peptides" which play the role of signal peptides promoting for instance cell proliferation. As previously mentioned, elastin peptides are part of matricin peptides.

Elastin peptides are considered as building blocks found in the natural elastin and have a short amino acid sequence. Examples of elastin peptides according to the invention are the penta-: KGGVG (SEQ ID N°30), VGGVG (SEQ ID N°31), GVGVP (SEQ ID N°32), VPGXG (SEQ ID N°33) (X being V, I, or K), hexa-:VGVAPG (SEQ ID N°34), hepta-: LGAGGAG (SEQ ID N°35) or nona-peptides: LGAGGAGVL(SEQ ID N°36).

In the context of the invention, a polypeptide or protein of elastin consists in repeated units of elastin, i.e. consists in repeated units (identical or different, preferably identical) of peptides of elastin and in particular of those (identical or different, preferably identical) of SEQ ID N°30 to 36.

"Derivatives" of elastin protein/polypeptide/peptide cover both elastin like proteins/polypeptides/peptides and proteins/polypeptides/peptides in which the amino acid sequence of the native elastin protein/peptide is mutated or contains one amino acid or more at its N- or C-terminus. The supplementary amino acids can be any amino acids.

Said mutations include deletions, substitutions, insertions and /or cleavage of one or more nucleic acids or amino acids.

Derivatives of elastin protein/polypeptide/peptide also covers derivatives of elastin like proteins/polypeptides/peptides.

The peptides, polypeptides and peptides of elastin and their derivatives, according to the invention, thus also cover elastin like peptides, polypeptides, peptides and their derivatives.

Elastin like proteins, polypeptides or peptides (ELP) are synthetic molecules comprising mainly several fold repeated units of peptides or derivatives.

There are many variants of elastin-like polypeptides, comprising repeated units of different elastin peptides, such as those of SEQ ID N°34 to 40 previously mentioned.

As an example, the elastin like peptides described in this invention is the hexapeptide VGVAPG of SEQ ID N°34 or its derivatives.

The elastin like polypeptide or polypeptide described in this invention can thus, for example, comprise a n-fold repeat of this hexapeptide (VGVAPG)ₙ or of its derivatives. The number n can be for example from 2 to 200, with a preference from 2 to 100.

In particular, derivatives of the peptide of SEQ ID N°34 contain the VGVAPG sequence with one or more amino acids at its N- and/or C-terminus.

In particular, the supplementary amino acids in the derivatives of the peptide of SEQ ID N°34 are an aspartic acid or a glutamic acid.

More particularly, said derivatives can be of SEQ ID N°37 (VGVAPGD) or SEQ ID N°38 (VGVAPGE).

Still particularly, the elastin like polypeptide described in this invention comprises a 4-fold repeat of the hexapeptide of SEQ ID N°34 (named in the present invention ELP4 and being of SEQ ID N° 39: VGVAPGVGVAPGVGVAPGVGVAPG). Another example of elastin like polypeptide derivative described in this invention comprises a repeat of the hexapeptide of SEQ ID N°38, more particularly a 4-fold of this hexapeptide (named in the present invention ELPE4 and being of SEQ ID N°40: VGVAPGEVGVAPGEVGVAPGEVGVAPGE).

An interesting feature of ELPs is that they can self-aggregates with an increase of temperature or a modification in pH and ionic strength.

The collagens are a superfamily of structurally related proteins that constitute essential building elements of connective tissues and participate to many biological functions in animals. These proteins exhibit a characteristic triple-helix tertiary structure resulting from the association of three polypeptide chains comprising repeated sequence Gly-Xaa-Yaa (GXY) where X and Y are frequently proline or 4-hydroxyproline involved in the triple helix formation.

In humans, there are at least 27 different types of collagens found in different tissues (as bones, bones, skin, tendon, blood vessels, eyes, etc....).

In the context of the invention, a recombinant peptide, polypeptide or protein of collagen consists in repeated units of collagen motifs, i.e in repeated units of the sequence GXY.

Derivatives" of collagen protein/polypeptide/peptide covers both collagen like proteins/polypeptides/peptides and proteins/polypeptides/peptides in which the amino acid sequence of the native collagen protein/polypeptide/peptide is mutated or contains one amino acid or more at its N- or C-terminus. This supplementary amino-acids can be any amino acids. Derivatives of collagen protein/polypeptide/peptide also covers derivatives of collagen like proteins/polypeptides/peptides.

Collagen like proteins, peptides or polypeptides comprise mainly several fold repeated units of collagen motifs, i.e of repeated units of the sequence GXY (whereas peptide, polypeptide or protein of collagen contain only those repeated units).

Said repeated units are called "collagen like domain" and are capable to form triple helix (as collagen proteins), as for instance, C-type lectins (collectins), which participate in the host defense mechanism.

Moreover, screening of genome databases of gene encoding collagen-like sequences containing repeated GXY motifs have identified gene in the genomes of bacteria and phages. However, these organisms seem to lack proline hydroxylases. Recent studies have shown that two recently identified streptococcal collagen-like proteins, Scl1 and Scl2 as models are capable of forming a stable triple helix without hydroxylation of the proline residues.

In particular, collagen like proteins, peptides or polypeptides are and/or derive (of one or more amino acids), from the CCMP2712 protein from *Guillardia theta.*

In one embodiment, derivatives according to the invention consists in an amino acids sequence at least 80% identical to the amino acids sequence of the recombinant peptide, polypeptide or protein according to the invention.

By "an amino acids sequence at least 80% identical to the amino acids sequence of (a)" is meant in particular, an amino acids sequence 81, 82, 85, 90, 91, 92, 93, 94, 95, 96, 97, 98 or 99% identical. By an amino acids sequence at least 95% "identical" to a query amino acids sequence of the present invention, it is intended that the amino acids sequence of the subject peptide, polypeptide or protein is identical to the query sequence except that the subject amino acids sequence may include up to five amino acids alterations per each 100 amino acids of the query amino acids sequence. In other words, to obtain an amino acids sequence at least 95% identical to a query amino acids sequence, up to 5% (5 of 100) of the amino acid residues in the subject sequence may be inserted, deleted, or substituted with another amino acid.

In the frame of the present application, the percentage of identity is calculated using a global alignment (*i.e.* the two sequences are compared over their entire length). Methods for comparing the identity of two or more sequences are well known in the art. The « needle » program, which uses the Needleman-Wunsch global alignment algorithm (Needleman and Wunsch, 1970 J. Mol. Biol. 48:443-453) to find the optimum alignment (including gaps) of two sequences when considering their entire length, may for example be used. The needle program is for example available on the ebi.ac.uk world wide web site. The percentage of identity in accordance with the invention is preferably calculated using the EMBOSS::needle (global) program with a "Gap Open" parameter equal to 10.0, a "Gap Extend" parameter equal to 0.5, and a Blosum62 matrix.

Amino acids sequences "at least 80%, 85%, 90%, 95% or 99% identical" to a reference sequence may comprise mutations such as deletions, insertions and/or substitutions compared to the reference sequence. In case of substitutions, amino acids sequences at least 80%, 85%, 90%, 95% or 99% identical to a reference sequence may correspond to a homologous sequence derived from another species than the reference sequence. In another preferred embodiment, the substitution preferably corresponds to a conservative substitution as indicated in the Table 1 below.

**Table 1**

| Conservative substitutions | Type of Amino Acid |
|---|---|
| Ala, Val, Leu, Ile, Met, Pro, Phe, Trp | Amino acids with aliphatic hydrophobic side chains |
| Ser, Tyr, Asn, Gln, Cys | Amino acids with uncharged but polar side chains |
| Asp, Glu | Amino acids with acidic side chains |
| Lys, Arg, His | Amino acids with basic side chains |
| Gly | Neutral side chain |

According to the invention, a nucleic acids sequence encoding said recombinant protein, polypeptide or peptide is a nucleic acids sequence encoding the proteins, peptides or polypeptides mentioned above.

In particular, for proteins, a nucleic acids sequence encoding elastin protein, elastin like proteins, collagen or collagen like proteins is contemplated, in particular, a collagen like protein is contemplated.

For example, the following nucleic acids sequences encoding collagen like proteins can be cited *Gtclp* (SEQ ID N°41), *Gtccld* (SEQ ID N°42) *or Gtcld* (SEQ ID N°43)..

In particular, for peptides, a nucleic acids sequence encoding collagen like peptides, collagen peptides, elastin peptides and elastin like peptides is contemplated; more particularly a nucleic acids sequence encoding an elastin like peptide or KTTKS peptides or KTTKS peptide derivatives.

For example, the following sequences in the Table 2 below can be cited.

**Table 2**

| Name of the peptide | Amino acid sequence | Nucleic acid Sequence |
|---|---|---|
| Elastin peptide VGVAPG | VGVAPG (SEQ ID N°34) | GTAGGTGTAGCTCCTGGT (SEQ ID N°44) |
| | | GTTGGTGTTGCTCCTGGA (SEQ ID N°45) |
| | | GTAGGTGTTGCTCCAGGT (SEQ ID N°46) |
| | | GTGGGTGTAGCTCCTGGT (SEQ ID N°47) |
| Elastin peptide VGVAPGE | VGVAPGE (SEQ ID N°38) | GTAGGTGTAGCTCCTGGTGAA (SEQ ID N°48) |
| | | GTTGGTGTTGCTCCTGGAGAA (SEQ ID N°49) |
| | | GTAGGTGTTGCTCCAGGTGAA (SEQ ID N°50) |
| | | GTGGGTGTAGCTCCTGGTGAA (SEQ ID N°51) |
| NY2 | KTTKS (SEQ ID N°18) | AAAACAACTAAATCA (SEQ ID N°52) |
| GNY2 | GKTTKS (SEQ ID N°19) | GGTAAAACAACTAAATCA (SEQ ID N°53) |

In particular, for polypeptides, a nucleic acids sequence encoding collagen like polypeptides, collagen polypeptides, elastin polypeptides and elastin like polypeptides are contemplated; more particularly a nucleic acids sequence encoding collagen polypeptide or an elastin like polypeptide or KTTKS polypeptide or KTTKS polypeptide derivatives.

For example, the following sequences in the Table 3 below can be cited.

**Table 3**

| Name of the polypeptide | Amino acid sequence | Nucleic acid Sequence |
|---|---|---|
| ELP4 | VGVAPGVGVAPGVGVAP GVGVAPG (SEQ ID N°39) | GTAGGTGTAGCTCCTGGTGTTGGTGTTGCTC CTGGAGTAGGTGTTGCTCCAGGTGTGGGTGT AGCTCCTGGT (SEQ ID N°54) |
| ELPE4 | VGVAPGEVGVAPGEVGV APGEVGVAPGE (SEQ ID N°40) | GTAGGTGTAGCTCCTGGTGAAGTTGGTGTTG CTCCTGGAGAAGTAGGTGTTGCTCCAGGTGA AGTGGGTGTAGCTCCTGGTGAA (SEQ ID N°55) |
| (NY3a)x5 | KTTKSDKTTKSDKTTKSD KTTKSDKTTKSD (SEQ ID N°26) | AAGACTACCAAAAGTGATAAAACAACTAAAAG CGATAAGACAACTAAATCTGATAAAACAACTA AATCAGACAAAACAACAAAATCAGAT(SEQ ID N°56) |
| G(NY3a)x5 | GKTTKSDKTTKSDKTTKS DKTTKSDKTTKSD (SEQ ID N°27) | GGTAAGACTACCAAAAGTGATAAAACAACTAA AAGCGATAAGACAACTAAATCTGATAAAACAA CTAAATCAGACAAAACAACAAAATCAGAT(SE Q ID N°57) |
| (NY3b)x5 | KTTKSEKTTKSEKTTKSE KTTKSEKTTKSE (SEQ ID N°28) | AAAACAACTAAATCAGAAAAAACTACAAAAAG TGAGAAGACTACAAAATCTGAGAAAACAACC AAGTCAGAAAAGACAACAAAATCAGAA(SEQ ID N°58) |
| G(NY3b)x5 | GKTTKSEKTTKSEKTTKS EKTTKSEKTTKSE(SEQ ID N°29) | GGTAAAACAACTAAATCAGAAAAAACTACAAA AAGTGAGAAGACTACAAAATCTGAGAAAACA ACCAAGTCAGAAAAGACAACAAAATCAGAA (SEQ ID N°59) |

Still particularly, nucleic acids sequence encoding derivatives according to the invention consists in a nucleic acid sequence at least 80% identical to the nucleic acid sequence encoding the recombinant peptide, polypeptide or protein according to the invention. By "a nucleic acids sequence at least 80% is meant in particular, a nucleic acids sequence 81, 82, 85, 90, 91, 92, 93, 94, 95, 96, 97, 98 or 99% identical . For example, a nucleic acids sequence 95% "identical" to a query sequence of the present invention, is intended to mean that the sequence of the polynucleotide is identical to the query sequence except that the sequence may include up to five nucleotide alterations per each 100 nucleotides of the query sequence. In other words, to obtain a polynucleotide having a sequence at least 95% identical to a query sequence, up to 5% (5 of 100) of the nucleotides of the sequence may be inserted, deleted, or substituted with another nucleotide. In other terms, the sequences should be compared on their entire length (i.e. by preparing a global alignment). For example, a first polynucleotide of 100 nt (nucleotides) that is comprised within a second polynucleotide of 200 nt is 50% identical to said second polynucleotide. The needle program, which uses the Needleman-Wunsch global alignment algorithm (Needleman and Wunsch, 1970, A general method applicable to the search for similarities in the amino acid sequence of two proteins, J. Mol. Biol. 48:443-453) to find the optimum alignment (including gaps) of two sequences when considering their entire length, may for example be used. Preferably, the percentage of identity in accordance with the invention is calculated using the needle program with a "Gap open" parameter equal to 10.0, a "Gap Extend" parameter equal to 0.5, and a Blosum 62 matrix. The needle program is for example available on the ebi.ac.uk World Wide Web site.

In one embodiment, the nucleic acids sequence encoding the protein, polypeptide or peptide comprising repeat units of amino acids according to the invention is codon optimized for expression in the chloroplast genome of the microalgae host cell.

By "recombinant peptide/polypeptide/protein" is meant in the art, and in the context of the present invention, an exogenous peptide/polypeptide/protein expressed from a recombinant gene (or recombinant nucleic acids sequence) i.e. an exogenous gene (or exogenous nucleic acids sequence) being from a different species (heterologous) or from the same species (homologous).

By "recombinant microalgae" is meant a microalgae comprising a nucleic acid sequence encoding a recombinant protein, polypeptide or peptide. In the context of the invention, the recombinant microalgae is transformed as further detailed below.

In the context of the invention, aprotinin and the recombinant protein, polypeptide or peptide are fused together by their N-terminus or C-terminus.

In one embodiment, said recombinant protein, polypeptide or peptide is fused to the C-terminus of aprotinin.

In order to identify and/or purify the recombinant protein, polypeptide or peptide according to the invention, an "epitope Tag" or "epitope Tag peptide" can be fused either to the N-terminus or the C-terminus of the fusion protein.

Accordingly, in one embodiment of the invention, the fusion protein according to the invention also comprises an epitope Tag, in particular a 3xFlag Tag.

In particular, said epitope Tag is placed at the N-terminus of the fusion protein. More particularly, another epitope Tag sequence can be placed at the C-terminus of the fusion protein according to the invention, alone or in addition to the one at the N-terminus and this, in order to monitor the release of the peptide/polypeptide/protein of interest to follow its cleavage, for example by an endoprotease.

Examples of epitope Tag sequences are Flag Tag (SEQ ID N°60: DYKDDDDK), 3xFlag Tag (SEQ ID N°61: DYKDDDDKDYKDDDDKDYKDDDDK), HA Tag (SEQ ID N°62: YPYDVPDYA), 3xHA Tag, His Tag (SEQ ID N°63: HHHHHH), which are described in the experimental part of the invention.

Still particularly, and as mentioned above, said fusion protein also comprises cleavage sites recognized by a specific protease.

Cleavage sites recognized by specific proteases are well known of the man skilled in the art. They are used to separate the aprotinin from the recombinant protein, polypeptide or peptide of interest, in the case the carrier should be removed if it could interfere with the activity or the structure of said protein, polypeptide or peptide and thus with its uses.

In particular, said cleavage sites are an endoprotease and/or endoproteinase recognition sequence (or protease cleavage site or protease recognition site). More particularly, the sequence of said cleavage sites is placed between the two coding sequences (the one of aprotinin and the one of the recombinant protein, polypeptide or peptide of interest according to the invention).

The cleavage of the fusion protein can be performed either *in vivo* (in the recombinant host cell before extraction or when apply on the skin for cosmetic peptides) or *in vitro* after extraction and purification by adding protease.

Non limitative examples of proteases are Factor Xa (FX), Tobacco Edge Virus protease (TEV), enterokinase (EK), SUMO protease, Thrombin, Human Rhinovirus 3C Protease (HRV 3C), endoproteinase Arg-C, endoproteinase Asp-C, endoproteinase Asp-N, endoproteinase Lys-C, endoproteinase Glu-C, proteinase K, IgA-Protease, Trypsin, chymotrypsin and Thermolysin.

Self-cleavage peptides can also be used, as for example the Intein system (Yang *et al.,* 2003), the viral 2A system (Rasala *et al.,* 2012) or the site of the preferredoxin from *Chlamydomonas* (Muto *at al.,* 2009).

In one embodiment, a linker can be placed between aprotinin and the protease cleavage site. Linkers can be classified into three types: flexible, rigid and cleavable. The usual function of linkers is to fuse the two partners of the fusion protein (e.g. flexible linkers or rigid linkers) or to release them under specific conditions (cleavable linkers) or to provide other functions of the proteins in drug design such as improving of their biological activities or their targeted delivery.

In one embodiment of the present invention, the linker can also make the protease cleaving site more accessible to the enzyme if necessary.

In one embodiment, the flexible linker contains small, non-polar (e.g. Gly) or polar (e.g. Ser or Thr) amino acids. Examples of such linkers are given in Chen *et al.,* 2013.

Flexible linkers according to the invention can be LG (SEQ ID N°64: RSGGGGSGGGGSGS) or LGM (SEQ ID N°65: RSGGGGSSGGGGGGSSRS).

The fusion protein according to the invention can be prepared as mentioned below and in particular as mentioned in the experimental part.

As previously mentioned, the present invention also relates to a method to produce a recombinant protein, polypeptide or peptide in algae, wherein said method comprises genetic transformation of algae with a nucleic acid sequence encoding a fusion protein, wherein said fusion protein comprises aprotinin and said recombinant protein, polypeptide or peptide.

A recombinant protein, polypeptide or peptide according to the invention is defined as mentioned previously.

An algae is as defined previously.

In particular, said method also comprises culturing algae. More particularly, algae is cultured in classic conditions known by the man skilled in the art. For example, *Chlamydomonas reinhardtii* is grown in TAP (Tris Acetate Phosphate) medium to mid-logarithmic phase (densities of approximately 1-2x10⁶ cell/ml), and/or at a temperature comprised between 23°C to 25°C (ideally 25°C), and/or on a rotary shaker in presence of constant light (70-150) µE/m²/s). The experimental part illustrates the conditions of culture.

A fusion protein is also as previously defined.

The method according to the invention comprises in particular:
(i) providing a nucleic acid sequence encoding a fusion protein, wherein said fusion protein comprises aprotinin and said recombinant protein, polypeptide or peptide;
(ii) introducing the nucleic acids sequence according to (i) into an expression vector which is capable of expressing the nucleic acid sequence in an algae host cell; and
(iii) transforming the genome of algae host cell by the expression vector.

In particular, said method further comprises:
(iv) identifying the transformed algae host cell;
(v) characterizing the microalgae host cell for the production of recombinant fusion protein;
(vi) extracting the recombinant fusion protein; and optionally
(vii) purifying the fusion protein.

More particularly, the method according to the invention allows to increase accumulation and/or stability and/or solubility and/or folding and/or activity of recombinant peptides, polypeptides or proteins comprising repeat units of amino acids in microalgae.

In particular, the steps (v) and (vi) comprise a step to extract proteins from algae. Said recovering step can be performed by any adapted method known by the person skilled in the art, such as a lysis (chemically, mechanically, thermally, enzymatically) followed by a step to eliminate the algae debris, performed by any adapted method known by the person skilled in the art, such as filtration, precipitation, centrifugation, etc.

In one embodiment, the method comprises a step (viii) in which the fusion protein is cleaved.

Said cleavage can be carried out by any method known by the man skilled in the art such as the use of suitable protease to release the recombinant peptide, polypeptide or protein.

Said step (viii) is optionally followed by another purification step (ix) of the recombinant protein, polypeptide or peptide.

In particular, said method further comprises an optional step (viii') between step (viii) and step (ix), in which the polypeptide is cleaved to allow the release of peptide units. Said cleavage can be carried out by any method known by the man skilled in the art such as the use of suitable endoproteinase.

In one embodiment, the recombinant protein, polypeptide or peptide obtained by the method according to the invention are chemically modified at their N- or C-terminus, for example by adding a palmitoyl group, an hydroxyl group, an alkoyl chain (i.e. an alkyl chain comprising an hydroxyl group), or a biotinyl group.

As mentioned above, the nucleic acids sequence according to the invention is introduced into an expression vector which is capable of expressing the nucleic acids sequence.

By "introducing the nucleic acids sequence into the expression vector" is meant cloning the nucleic acids sequence encoding the recombinant protein/polypeptide/peptide inside the expression vector with methods well known by the skilled man and in the way to lead to the expression of this nucleic acid sequence.

"Expression vector" or "transformation vector" or "recombinant DNA construct", or similar terms, are defined herein as DNA sequences that are required for the transcription of recombinant genes and the translation of their mRNAs in the algae host cells. "Transformation vectors" contains one or more expression cassettes for the expression of recombinant genes (one or more gene encoding the protein, peptide or polypeptide of interest and often a selectable marker). In the case of chloroplast genome transformation, expression vectors also contain homologous recombination regions for the integration of expression cassettes inside the chloroplast genome.

In the context of the invention, the expression vector can be in particular a circular molecule with a plasmid backbone containing the two homologous recombination regions and flanking the expressions cassettes, or a linearized molecule corresponding to the expression vector linearized by enzymatic digestion or to a PCR fragment containing only the expression cassettes flanked by the two homologous recombination regions.

In particular, expression vectors of the invention comprise at least one expression cassette and are for example vectors pAU76, pAU97, pAU94, pLA01, pLA02, pLA03, pLA04, pNY18, pNY19, pNY13, pNY14, pNY15 or pNY16.

"Expression cassette" contains a coding sequence fused operationally to one or more regulatory elements or regulatory sequences, as for instance, fused at its 5'end to a promoter and/or 5'UTR and/or at its 3'end to a 3'UTR.

The "coding sequence" is the portion of a gene and of its corresponding transcribed mRNA which is translated into the recombinant protein/polypeptide/peptide. The coding sequence includes, for example and a translation initiation control sequence and a stop codon. In some embodiment, the expression cassette can contain a polycistron composed of more than one coding sequence encoding several proteins under the control of only one promoter/5'UTR and 3'UTR.

Said expression cassettes are flanked by left (LHRR) and right (RHRR) endogenous sequences identical to those surrounding the targeted integration site into the chloroplast genome. These left (LHRR) and right (RHRR) homologous regions allow the integration of expression cassettes after homologous recombination exchange between the regions of homology.

Homologous recombination is the ability of complementary DNA sequences to align and exchange regions of homology. Transgenic DNA ("donor") containing sequences homologous to the genomic sequences being targeted ("template") is introduced into the organism and then undergoes recombination into the genome at the site of the corresponding genomic homologous sequences.

By its very nature homologous recombination is a precise gene targeting event, hence, most transgenic lines generated with the same targeting sequence will be essentially identical in terms of phenotype, necessitating the screening of far fewer transformation events.

In the case of chloroplast genome transformation of microalgae, the integration of expression cassettes inside the chloroplast genome occurs after homologous recombination between the endogenous homologous sequences of the expression vector with the genome sequences identical or similar to those surrounding the targeted integration site into the chloroplast genome.

In the context of the present invention, other integration sites can be, between the genes *rbcL* and *atpA,* or *psaB* and *trnG,* or *atpB* and *16S rDNA,* or *psaA exon3* and *trnE,* or *trnE* and *psbH* or *psbN* and *psbT,* or *psbB* and *trnD.*

In some embodiments, in order to enhance its accumulation, the recombinant protein or polypeptide or peptide can be fused to endogenous proteins, as for instance to the large subunit of ribulose bisphosphate carboxylase (Rubisco LSU). In this case, the promoter and 5'UTR will be those of the endogen *rbcL* gene, after homologous recombination of the transformation vector into the chloroplast genome.

The protein, peptide or polypeptide of interest will be further separated from RBCL either *in vivo* or *in vitro,* depending of the chosen processing system.

In one embodiment, said coding sequence in the expression cassettes also comprises a nucleic acids sequence encoding an epitope Tag, in particular the Flag epitope Tag, more particularly the Flag Tag repeat 3 times (3xFlag Tag), in order to identify and/or purify the recombinant protein, polypeptide or peptide.

In particular, said epitope Tag sequence is placed at the N-terminus of the protein, peptide or polypeptide. More particularly, another epitope Tag sequence can be placed at the C-terminus of the protein, peptide, or polypeptide according to the invention, alone or in addition to the one at the N-terminus and this, in order to monitor the release of the peptide/polypeptide/protein of interest to follow its cleavage, for example by an endoprotease.

Examples of epitope Tag sequences are as previously mentioned.

In one embodiment, said coding sequence in the expression cassettes comprises nucleic acid sequence encoding not only the said fusion protein but also an amino acid sequence allowing the production of the said fusion protein in specific cell compartment.

"Promoter" as used herein, refers to a nucleic acid control sequence that directs transcription of a nucleic acid.

"5'UTR" or "5' untranslated region" is the region of a mRNA that is upstream from the initiation codon.

"3'UTR" or "3' untranslated region" is the section of mRNA that follows the translation termination codon.

5'UTRs and 3'UTRs are required for transcript (mRNA) stability and translation initiation.

For *Chlamydomonas reinhardtii* nuclear expression, promoters, 5'UTRs and 3'UTRs that can be used in the context of the invention are for example: the *psaD* promoter and 5'UTR, the chimeric promoter containing the *C. reinhardtii hsp70A* promoter fused to the *C. reinhardtii rbcS2* promoter followed by the first intron of the *C. reinhardtii rbcS2* gene. 3'UTRs can be the 3'UTRs from *C. reinhardtii rbcS2* or *psaD* genes.

For *Chlamydomonas reinhardtii* chloroplast expression, promoters, 5'UTRs and 3'UTRs that can be used in the context of the invention are for example: the *psbD* promoter and 5'UTR (SEQ ID N°66), the *psbA* promoter and 5'UTR, the *psaA* promoter and 5'UTR, the *atpA* promoter and 5' UTR, the *atpB* promoter and 5' UTR, the *16S rRNA* promoter (*Prrn*) promoter fused with the *atpA* 5'UTR (SEQ ID N°67), the *psbA* 3' UTR, the *atpA* 3'UTR (SEQ ID N°68) or the *rbcL* 3' UTR (SEQ ID N°69).

A 5'UTR from exogenous origin as for instance the 5'UTR of the gene *10L* of the bacteriophage T7 can be used also fused downstream a *Chlamydomonas* promoter. In particular, the nucleic acid sequence is operationally linked at its 5'end to the *Chlamydomonas reinhardtii 16S rRNA* promoter (*Prrn*).

Stable expression and translation of the gene of interest according to the present invention can for example be controlled by the promoter and 5'UTR from *psbD* and the *atpA* 3'UTR.

In one embodiment, the promoter less gene encoding the protein of interest can be integrated after homologous recombination region inside the chloroplast genome just downstream a native promoter.

As mentioned above, the chloroplast genome of microalgae host cell is transformed by the expression vector.

Genetic transformation of algae according to the invention, and more particularly of a microalgae according to the invention, and even more particularly of the chloroplast genome of photosynthetic host cells by chloroplast transformation vector can be carried out according to any suitable techniques well known by the man skilled in the art including, without limitations biolistics (Boynton *et al.,* 1988; Goldschmidt-Clermont, 1991), electroporation (Fromm et al., Proc. Nat'l. Acad. Sci. (USA) (1985) 82:5824-5828 ; see Maruyama et al. (2004), Biotechnology Techniques 8:821-826), glass bead transformation (Purton *et al.,* revue), protoplasts treated with CaCl₂ and polyethylene glycol (PEG) (see Kim et al. (2002), Mar. Biotechnol. 4:63-73) or microinjection.

In particular, said genetic transformation uses the helium gun bombardment technique of gold micro-projectiles complexed with transforming DNA.

Host cells according to the invention are cells of algae, and more particularly of microalgae.

To select the algal transformants, a selectable marker gene may be used. Mention may be made for example of the *aadA* gene coding aminoglycoside 3"-adenylyltransferase and conferring the resistance to spectinomycin and streptomycin in the case of *Chlamydomonas reinhardtii* chloroplast genome transformation. In another embodiment, the selectable marker gene can be the *Acinetobacter baumannii aphA-6 Ab* gene encoding 3'-aminoglycoside phosphotransferase type VI and conferring the kanamycin resistance.

For algal nuclear genome transformation and in particular for *Chlamydomonas reinhardtii,* the selectable marker gene may be the *aphVIII* gene coding an aminoglycoside 3'-phosphotransferase conferring the resistance to paromomycin, kanamycin and neomycin.

Chloroplast genome engineering can thus be performed using selectable maker gene conferring resistance to antibiotic or using rescue of photosynthetic mutant.

The nucleic acid sequence encoding a fusion protein of the present invention comprises a nucleic acid sequence encoding the aprotinin and a nucleic acid sequence encoding a protein, polypeptide or peptide according to the invention.

These nucleic acid sequence are as defined previously.

In one embodiment, said recombinant protein, polypeptide or peptide is fused to the C-terminus of aprotinin.

In order to identify and/or purify the recombinant protein, polypeptide or peptide according to the invention, an epitope Tag peptide can be fused either to the N-terminus or the C-terminus of the fusion protein.

As previously mentioned, in one embodiment of the invention, the fusion protein according to the invention also comprises an epitope Tag sequence and/or still particularly, and as mentioned above, said fusion protein also comprises a signal peptide and one ore more protease cleavage site.

In particular, the nucleic acid sequence encoding aprotinin is operationally fused in its 5'end to a nucleic acid sequence for the HA Tag peptide followed by a signal peptide (HA-SP), the 3XFlag Tag peptide and the recognition site of Factor Xa protease (named HA-SP-3F-FX-APRO or chimeric aprotinin of SEQ ID N°70 and corresponding nucleic acid sequence of SEQ ID N°71).

In particular, said protease cleavage site is an endoprotease and/or an endoproteinase recognition sequence. More particularly, the sequence of said protease cleavage site is placed between the two coding sequences (the one of aprotinin and the one of the recombinant protein, polypeptide or peptide of interest according to the invention).

In one embodiment, a linker can be placed between aprotinin and the cleavage site. Linkers can be classified into three types: flexible, rigid and cleavable. The usual function of linkers is to fuse the two partners of the fusion protein (e.g. flexible linkers or rigid linkers) or to make the protease cleaving site more accessible to the enzyme if necessary.

Characterization of the microalgae host cell producing the fusion protein and/or recombinant protein, polypeptide or peptide can be conducted by techniques known by the man skilled in the art, for example by PCR screening of the antibiotic resistant transformants or Western Blot analysis performed on total protein extracts.

In one embodiment, it comprises an extraction step or extraction step and a purification step.

Extraction of total proteins can be carried out using well known techniques (centrifugation, lysis, sonication,...).

Identification of fusion or recombinant proteins can be carried out by Western blot using specific antibodies.

Purification can be carried out using well-known techniques. In one embodiment, it comprises an affinity chromatography and/or a step of separation of the peptide, polypeptide or protein according to the invention from aprotinin (for example by enterokinase protease digestion) and/or a size exclusion chromatography.

In one embodiment, the step of affinity chromatography can be replaced by an ion exchange chromatography, less expensive for large scale purification.

The invention will be further illustrated by the following figures and examples.

### FIGURES

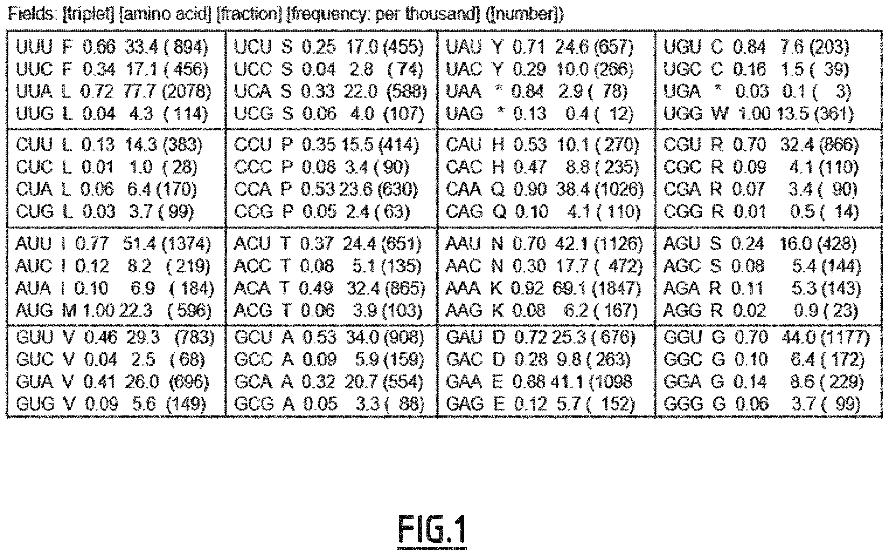

**Figure 1****:** Codon usage in the *Chlamydomonas reinhardtii* chloroplast genome.

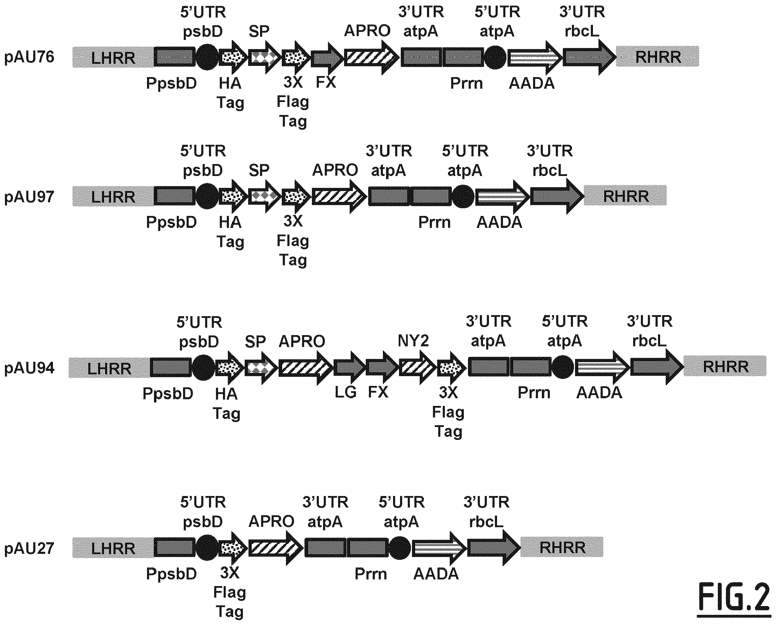

**Figure 2****:** Schematic presentation of the chloroplast transformation vectors for aprotinin production.

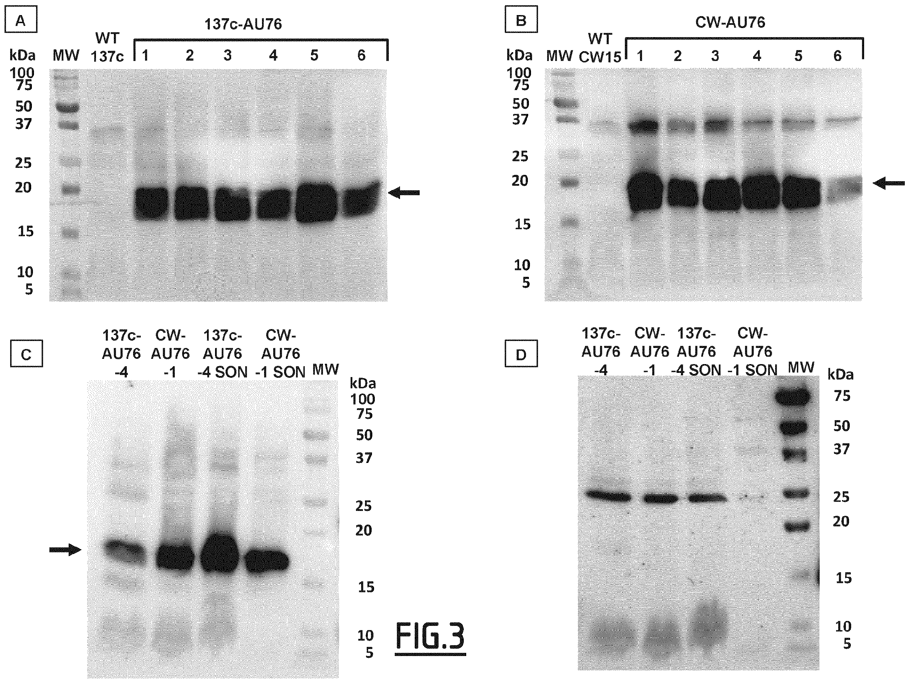

**Figure 3****:** Western blot analysis of independent algae transformants 137c-AU76 and CW-AU76 expressing chimeric aprotinin from the algae chloroplast genome, using monoclonal anti-Flag M2 (A, B, C) or anti-HA (D) antibodies. 50 µg of each total soluble protein samples extracted with SDS buffer lysis were separated on a 15% SDS polyacrylamide gel. SON (C and D): total soluble proteins extracted by sonication and loaded on a 15% SDS polyacrylamide (85 µg for 137c-AU76-4 SON and 33 µg for CW-AU76-1 SON). MW: molecular weight standard. Arrows indicate the positions of recombinant proteins.

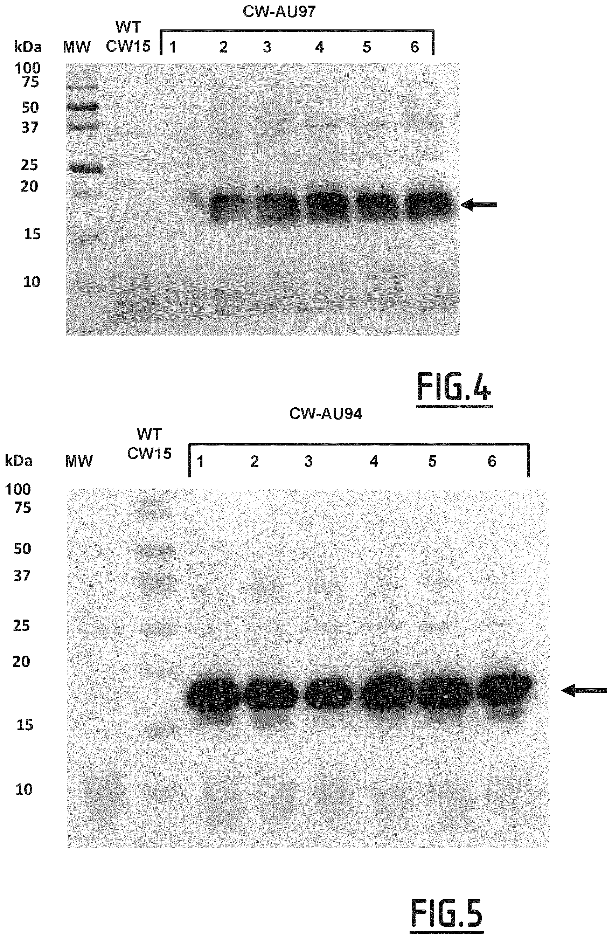

**Figure 4****:** Western blot analysis of independent algae transformants CW-AU97 expressing chimeric aprotinin from the algae chloroplast genome, using monoclonal anti-Flag M2 antibody. 50 µg of each total soluble protein samples extracted with SDS buffer lysis were separated on a 15% SDS polyacrylamide gel. MW: molecular weight standard. Arrows indicate the positions of recombinant proteins.
**Figure 5****:** Western blot analysis of independent algae transformants CW-AU94 expressing chimeric aprotinin from the algae chloroplast genome, using monoclonal anti-Flag M2 antibody. 50 µg of each total soluble protein samples extracted with SDS buffer lysis were separated on a 15% SDS polyacrylamide gel. MW: molecular weight standard. Arrows indicate the positions of recombinant proteins.

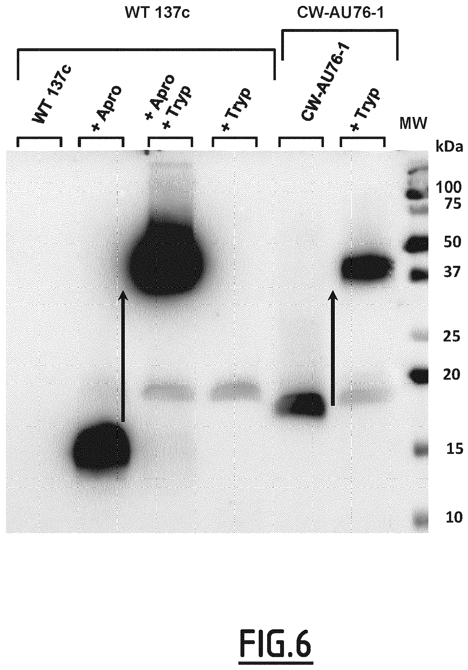

**Figure 6****:** Western blot analysis using monoclonal anti-aprotinin antibody and the trypsin binding assays performed on total soluble protein extracts from WT 137c (50 µg) in presence or not of aprotinin standard (+ Apro: 100 ng) or from CW-AU76-1 (25 µg). In some assay, 1.8 µg of trypsine was added (+ Tryp). Samples (not heat denaturated) were separated on a 15% SDS polyacrylamide gel under non reducing conditions.

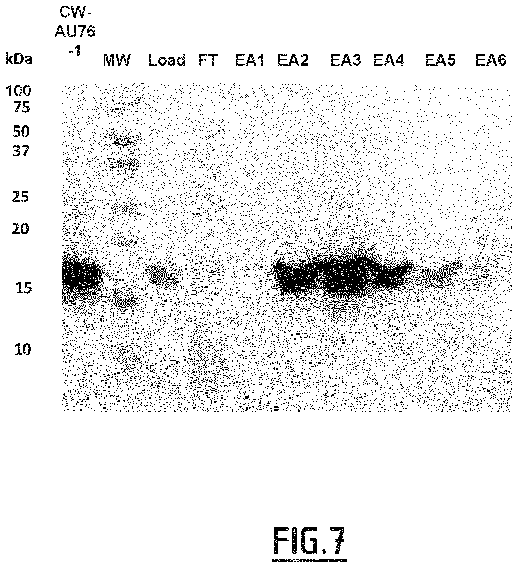

**Figure 7****:** Western Blot analysis of different elution fractions from anti-Flag M2 affinity chromatography performed on a protein extract from CW-AU76-1 and using monoclonal anti- Flag M2 antibody. Protein samples (30 µg of total soluble proteins extracted by sonication wild type CW15 or 25 µl of elution (EA) or flow through (FT) fractions) were loaded on a 15% SDS polyacrylamide gel. MW: molecular weight standard. Load: total soluble protein extracted by sonication before the incubation with anti- anti-Flag M2 resin.
Arrows indicate the positions of purified recombinant proteins.

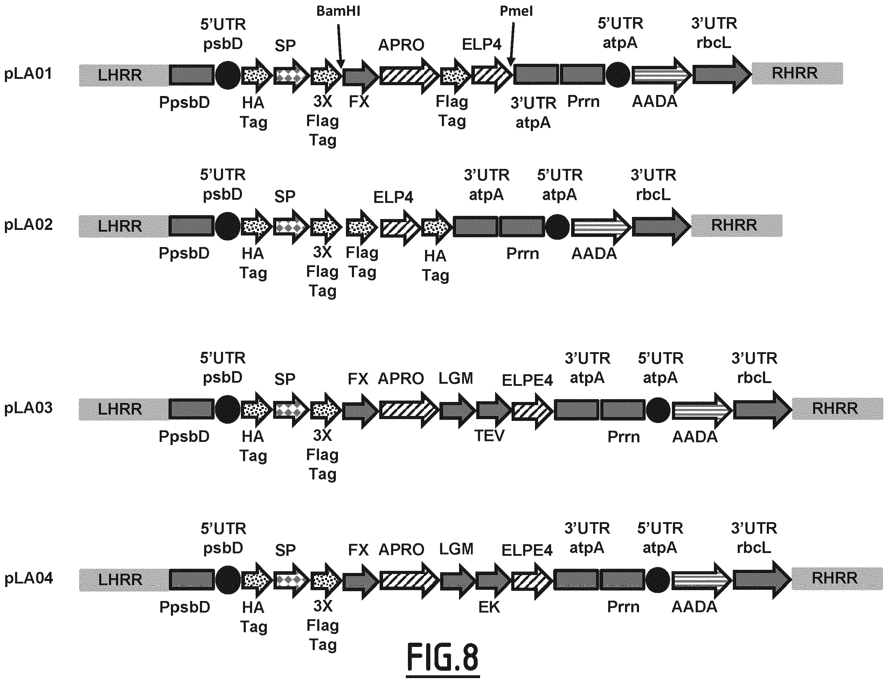

**Figure 8****:** Schematic presentation of the chloroplast transformation vectors for elastin polypeptide and peptide production.

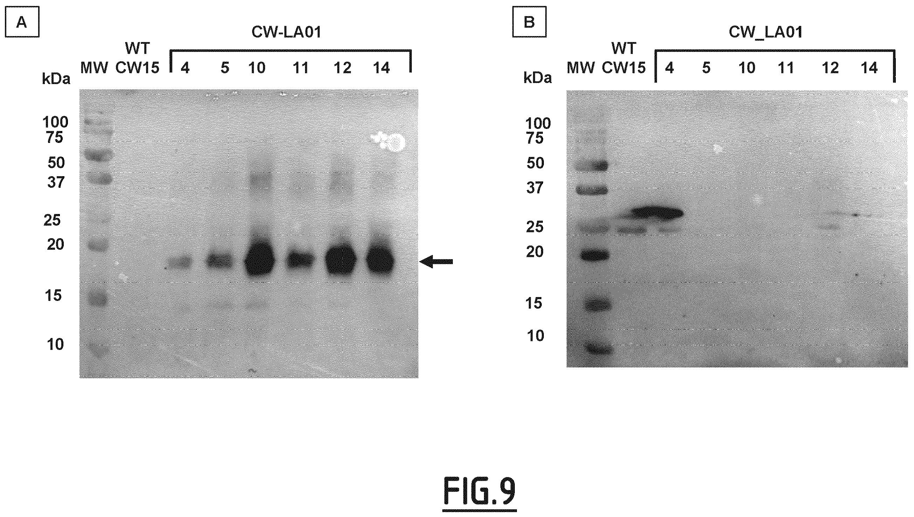

**Figure 9****:** Western blot analysis of algae cells transformed with pLA01, using monoclonal anti-Flag (A) or anti-HA (B) antibodies. 50 µg of total soluble protein samples extracted with SDS buffer lysis from wild type (WT) CW15 cells and from independent CW-LA01 transformants were separated on a 15% SDS polyacrylamide gel. MW: molecular weight standard. Arrows indicate the positions of recombinant proteins.

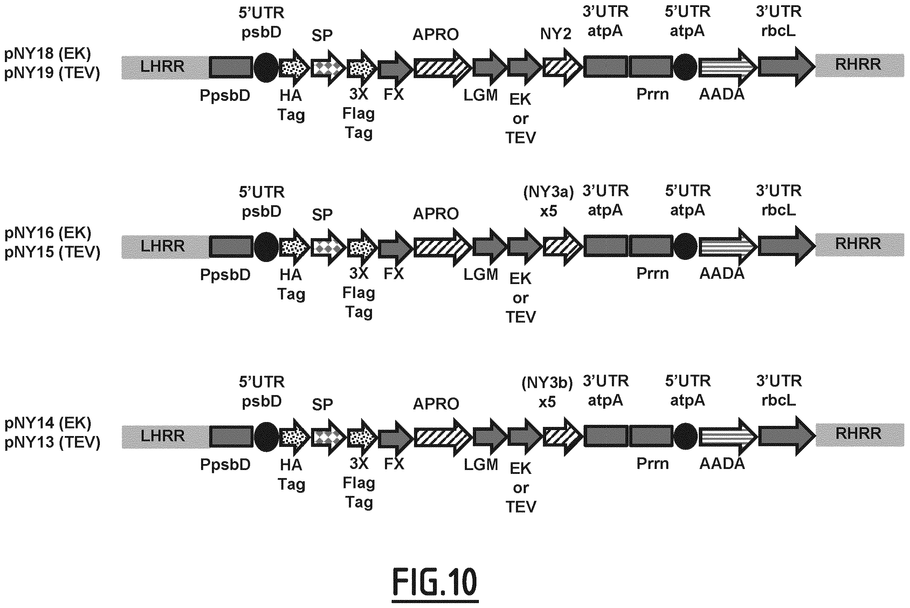

**Figure 10****:** Schematic presentation of the chloroplast transformation vectors for the production of fusion proteins with peptides and polypeptides of KTTKS and their derivatives.

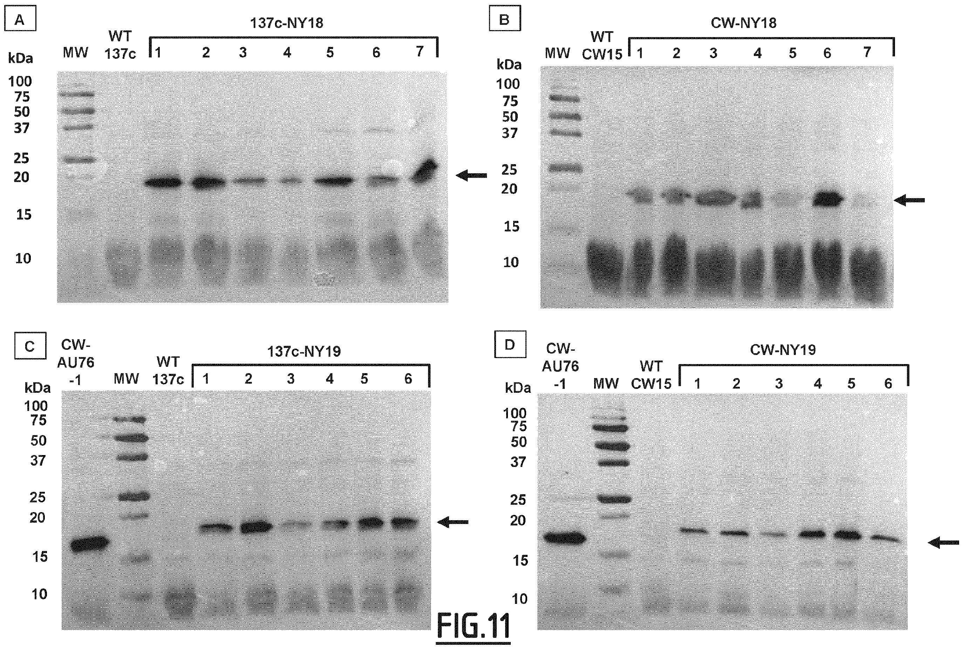

**Figure 11****:** Western blot analysis of independent algae transformants 137c or CW-NY18 (A, B) and 137c or CW-NY19 (C, D) expressing the genes encoding the fusion proteins containing the peptide, using monoclonal anti-Flag M2 antibody. 100 µg or 50 µg of each total soluble protein samples extracted with SDS buffer lysis from NY18 or NY19, respectively, were separated on a 15% SDS polyacrylamide gel. MW: molecular weight standard. 50 µg of total soluble protein samples extracted by sonication from CW-AU76-1 transformant was loaded as positive control (C, D). Arrows indicate the positions of recombinant proteins.

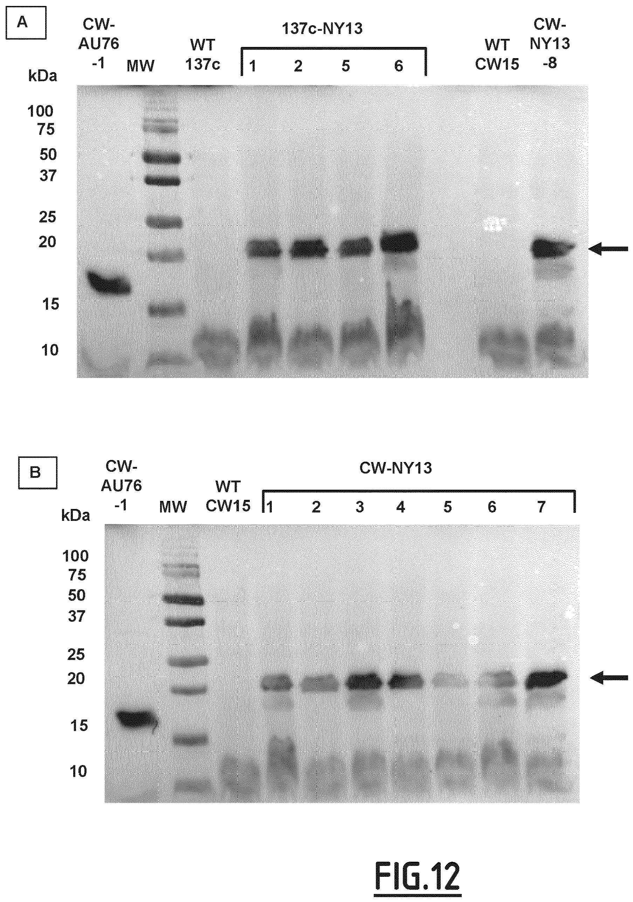

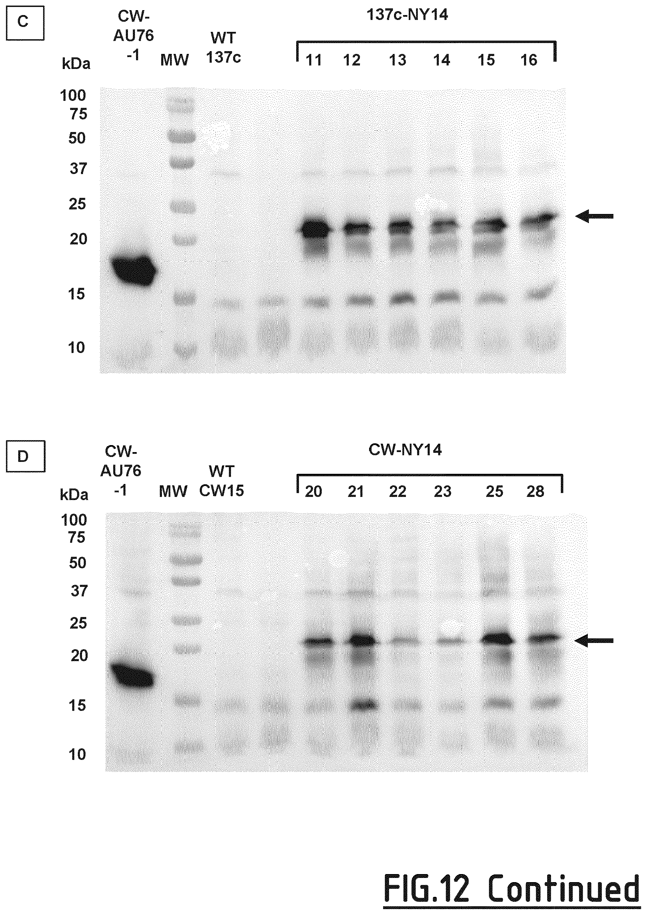

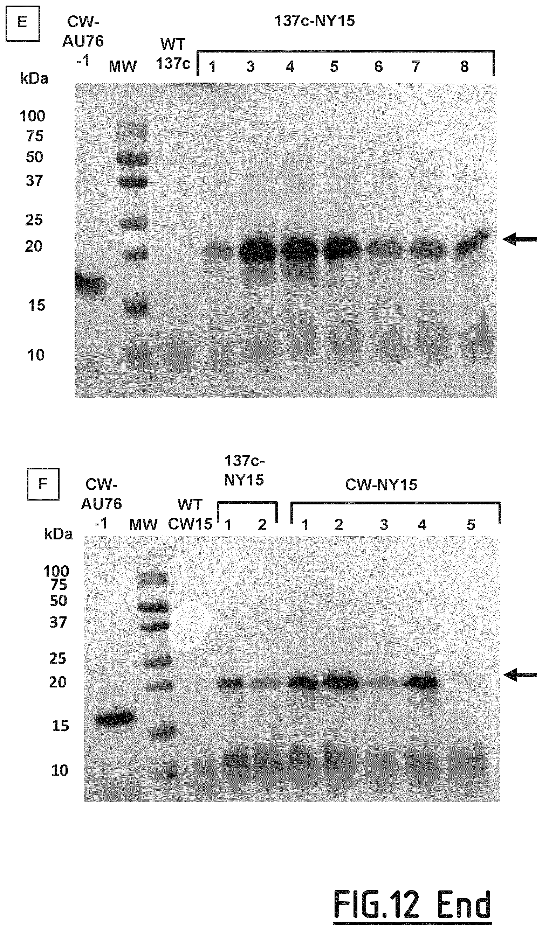

**Figure 12****:** Western blot analysis of independent algae transformants 137c or CW-NY13 (A, B), 137c or CW-NY14 (C, D) and 137c or CW-NY15 (E,F) expressing the genes encoding the fusion proteins containing the polypeptide (NY3a)x5 and (NY3b)x5, using monoclonal anti-Flag M2 antibody. 100 µg (or 50 µg for NY14 transformants) of each total soluble protein samples extracted with SDS buffer lysis from NY13 or NY15, respectively, were separated on a 15% SDS polyacrylamide gel. MW: molecular weight standard. 50 µg of total soluble protein samples extracted by sonication from CW-AU76-1 transformant was loaded as positive control. Arrows indicate the positions of recombinant proteins.

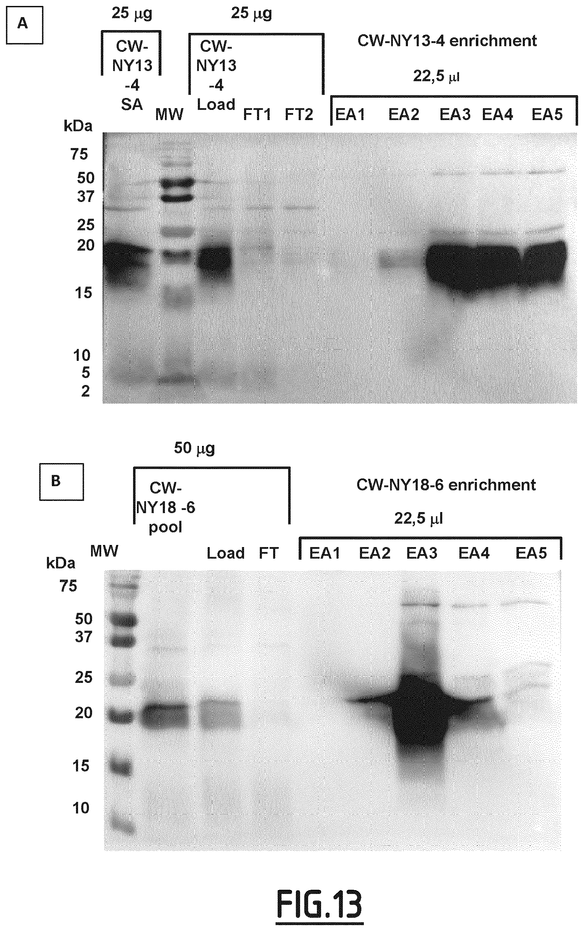

**Figure 13****:** Western Blot analysis of different elution fractions from anti-Flag M2 affinity chromatography performed on a protein extract from CW-NY13-4 (A) and CW-NY18-6 (B) transformants using monoclonal anti-Flag M2 antibody. Different quantities (25 or 50 µg) of protein samples extracted by sonication and/ or precipitated by ammonium sulfate or volume of elution fraction were loaded on a 15% SDS polyacrylamide gel. A) CW-NY13-4 SA: precipitated proteins by ammonium sulfate from CW-NY13-4. MW: molecular weight standard. Load: total soluble protein extracted by sonication before the incubation with anti-Flag M2 resin. FT: Flow through. EA: elution fraction. W: wash fraction. Arrows indicate the positions of purified recombinant proteins.

### EXAMPLES

### Example 1

### Material and Methods

All oligonucleotides and synthetic genes were purchased from Eurofins. All enzymes were purchased from NEB, Promega, Invitrogen and Sigma Aldrich/Merck. All plasmids were built on the pBluescript II backbone.

### Algal strains and growth conditions

The two algal strains used are the *Chlamydomonas reinhardtii* wild type (137C; mt+) and the cell wall deficient strain CW15 (CC-400; mt+), obtained from the *Chlamydomonas* Resource Center, University of Minnesota).

Prior to transformation, all strains were grown in TAP (Tris Acetate Phosphate) medium to mid-logarithmic phase (densities of approximately 1-2x10⁶ cell/mL) at a temperature comprised between 23°C to 25°C (ideally 25°C) on a rotary shaker in presence of constant light (70-150) µE/m²/s).

Transformants were grown in the same conditions and the same media supplemented with (containing) 100 µg/mL of spectinomycin or 100 µg/mL kanamycin, depending of the selectable marker gene present in the transformation vector.

Growth kinetics was also followed by measuring the optical density at 750 nm using a spectrophotometer.

### Algal transformation

*Chlamydomonas reinhardtii* cells were transformed using the helium gun bombardment technique of gold micro-projectiles complexed with transforming DNA, as described in the article Boynton *et al.,* 1988. Briefly, the *Chlamydomonas reinhardtii* cells were cultivated in TAP medium until midlog phase, harvested by gentle centrifugation, and then resuspended in TAP medium to a final concentration of 1.10⁸ cells/mL. 300 µL of this cell suspension was plated onto a TAP agar medium supplemented with 100 µg/mL of spectinomycin or 100 µg/mL of kanamycin, depending of the selectable marker gene present in the transformation vector. The plates were bombarded with gold particles (S550d; Seashell Technology) coated with transformation vector, as described by the manufacturer. The plates were then placed at 25°C under standard light conditions to allow selection and formation of transformed colonies.

### Total DNA extraction and PCR screening of positive transformants

Total DNA extraction was performed using the chelating resin Chelex 100 (Biorad) from single colonies (with size of around 1 mm in diameter) of wild type and/or antibiotic resistant transformants *Chlamydomonas* strains.

From isolated colonies, a quantity of cells corresponding to about 0.5 mm in diameter was removed with a pick and resuspended in 20 µL of H₂O. 200 µL of ethanol were added and incubated 1 min at room temperature. 200 µL of 5% Chelex were incorporated and vortexed. After an incubation of 8 min at 100 °C, the mixture was cooled down and centrifuged 5 min at 13,000 rpm. Finally, the supernatant was collected.

After transformation, algae colonies growing onto restrictive solid medium plates were expected to have the antibiotic resistant gene incorporated the transgene(s) into their genome.

In order to identify stable integration of the recombinant genes into the algal genome of the antibiotic resistant transformants were screened by Polymerase Chain Reaction (PCR or PCR amplification) in a thermocycler using 1 µL of total DNA previously extracted as template, two synthetic and specific oligonucleotides (primers) and Taq polymerase (GoTaq, Promega). The cycles of PCR amplification will followed the guidelines recommended by the manufacturer. The PCR reactions were subjected to gel electrophoresis in order to check the PCR fragment of interest.

### Protein extraction, Western blot analyses

*Chlamydomonas* cells (50 mL, 1-2.10⁶ cells/mL) were collected by centrifugation. Cell pellet was resuspended in lysis buffer (50 mM Tris-HCI pH 6.8, 2% SDS, 10 mM EDTA). In some embodiments of the example, the lysis buffer didn't contain 10 mM EDTA. After 30 min at room temperature, cell debris were removed by centrifugation at 13000 rpm and the supernatant containing the total soluble proteins was collected.

Depending on the further analysis step, total soluble proteins were extracted under non denaturing conditions. Cell pellet was resuspended in a buffer containing 50 mM Tris-HCI (pH 6.8 or 8) or 20 mM Tris-HCI (pH 6.8 or 8). The sonication step was carried out with the algal cell suspension held on ice, using a cell disruptor a sonicator FB505 500W (Sonic/FisherBrand) and a micro-tip setting of 20 % power, with continuous sonication for 5 min. After sonication, cell debris were removed by centrifugation at 13000 rpm, 30 min.

Total soluble proteins present in the supernatant were quantified using the Pierce BCA protein assay kit, following the instructions of the supplier (Thermofisher).

Total soluble protein samples (50 or 100 µg or another quantity further mentioned in the example depending of the experiment) were separated in a 12 or 15% Tris-glycine SDS-PAGE prepared according to Laemmli (1970).

For experiments performed under reducing conditions, samples were prepared in Laemmli sample loading buffer with 50 mM DTT (or more depending of the fusion protein) or 5% Beta-mercaptoethanol, and further denaturated 5 min at 95°C before loading. The SDS PAGE experiments were carried out using a Protein gel tank from BioRad.

After separation, samples were blotted onto a nitrocellulose membrane (GE HealthCare) using standard transfer buffer and a Trans-Blot® Turbo™ Transfer System from Biorad. In order to visualize the transferred proteins, the nitrocellulose membrane were stained by Ponceau S dye. Membranes were further blocked with Tris-buffered saline Tween buffer (TBS-T) (50 mM Tris-HCI pH 7.5, 150 mM NaCl, 0.1% Tween-20) containing 5% Bovin Serum Albumin (BSA). After one hour of saturation at room temperature under gently shaking, membranes were incubated during one night at 4°C with TTBS buffer containing mouse primary antibody (See Table 1).

The antibody mentioned in Table 1 below were used as primary antibodies.

**Table 1: primary antibodies**

| Primary antibody | Source | Dilution |
|---|---|---|
| Monoclonal ANTI-FLAG® M2 antibody produced in mouse | Sigma | 1:1000 |
| Monoclonal ANTI-HA PURIFIED antiobody produced in mouse IGG | Sigma | 1:6000 |
| Monoclonal ANTI-Aprotinin antibody produced in mouse | Abcam | 1:2000 or 1:3000 |

After three_washes with TBS-T-BSA buffer, membranes were incubated one hour at room temperature with TBS-T-BSA buffer containing secondary antibodies (Promega). After four washes with TTBS buffer and one wash with TBS buffer, the membranes were revealed with a linked secondary antibody conjugated to horseradish peroxidase (HRP) (Anti-Mouse IgG (H+L), HRP Conjugate; Promega,) and visualized by enhanced chemiluminescence (ECL) using Clarity Max as ECL substrate (Biorad) and the ChemiDoc™ XRS+ system (Biorad).

### Protein purification

Depending on the protein and on the further steps to which the protein is submitted, the first or second method below is conducted.
1) After centrifugation, algae cell pellets were resuspended in a buffer containing 50 mM Tris-HCI pH8, 500 mM NaCl and 0.1% Tween 20. Approximately, 10 mL of buffer were used per g of wet algal cells, depending of the transformants. The resuspended cells were sonicated in the same conditions as previously described.
2) After centrifugation, algae cell pellets were resuspended in a buffer containing 20 mM Tris-HCI pH 8. The resuspended cells were sonicated in the same conditions as previously described. The total soluble proteins were precipitated with 80% ammonium sulfate as described by Wingfield, 2001. After a 15000 g centrifugation for 30 min at 4°C, the protein pellets were resolubilized in 60 mM Tris-HCI pH8 buffer. These suspensions were dialysed in Slide-A-Lyzer Dialysis Cassettes (3.5 kDa MWCO, Thermo Scientific) as described by the manufacturer against the previous buffer. The next step being an anti-FLAG M2 affinity chromatography, NaCl and Tween 20 were added to the dialyzed samples to adjust the buffer composition to that of the binding buffer hereinafter described.

### Affinity chromatography

All recombinant proteins were tagged in their N-terminal with a Flag-Tag epitope which will bind specifically on an anti-Flag M2 affinity gel (Sigma/Merck). This resin contains a mouse monoclonal Anti-Flag® M2 antibody that is covalently attached to agarose.

All steps of this experiment were carried out as described by the manufacturer. Briefly, the samples of total soluble proteins were filtered using a cellulose acetate 0.45 µm filter and mixed with anti-Flag® M2 affinity gel prepared as recommended by the manufacturer and equilibrated in binding buffer (50 mM Tris-HCI pH8, 500 mM NaCl, 0.1% Tween 20). Approximately, 1 mL of resin was used per 4 to 8 g of wet algal cells, depending of the transformants. Binding of the recombinant fusion protein was performed at 4°C for 4h or overnight with a gently and continuous end-over-end mixing. After incubation, the mixture of soluble protein incubated with resin were loaded by gravity on an empty Bio-rad Econo-pac column or collected by centrifugation, and washed several times with 40 column volumes of TBST and 20 column volumes of TBS. The protein of interest was eluted from the resin using 100 mM Glycine pH 3.5, 500 mM NaCl and neutralized with Tris-HCI pH 8 to a final concentration of 50 mM. Each elution fraction were further analyzed by SDS-PAGE and Western Blot.

The elution fractions containing the protein of interest were dialyzed in Slide-A-Lyzer Dialysis Cassettes (3.5 kDa MWCO, Thermo Scientific) as described by the manufacturer against the buffer used in the further step, as for instance, for the protease digestion. The dialyzed samples were concentrated using Vivaspin 6 (3 kDa MWCO, GE Healthcare).

### Separation of the protein of interest from the carrier

The separation of the protein of interest from the carrier was made by protease digestion, in particular, in the present invention by enterokinase (light chain) or Tobacco Etch Virus (TEV) Protease from New England BioLabs (NEB).

Enzymatic digestions were performed as recommended by the manufacturer.

For example, for enterokinase light chain digestion, reactions combined 25 µg of protein of interest in 20 µL of buffer (20 mM Tris-HCI pH 8.0, 50 mM NaCl, 2 mM CaCl₂), with 1 µL of enterokinase light chain. Incubation was made at 25°C for 16h.

For example, for TEV digestion, typical reaction recommended by the manufacturer combined 15 µg of protein substrate with 5 µL of TEV protease reaction buffer (10X) to make a 50 µL total reaction volume. After addition of 1 µL of TEV Protease, reaction was incubated at 30°C for 1 hour or 4°C overnight.

For example, for Factor Xa digestion, the manufacturer recommended to digest 50 µg of fusion protein with 1 µg of FXa in a volume of 50 µL at 23°C for 6h. The buffer reaction consisted in 20 mM Tris-HCI pH 8.0, 100 mM NaCl and 2 mM CaCl₂.

### Clivage of the polypeptide by endoproteinases

The choice of the endoproteinase used to cleave the polypeptide of interest depends of the amino acid sequence of this polypeptide. Endoproteinases can be for instance, endoproteinase Glu-C, endoproteinase Arg-C, endoproteinase Asp-C, endoproteinase Asp-N, or endoproteinase Lys-C.

Enzymatic digestions were performed as recommended by the manufacturer. For example, for endoproteinase Glu-C digestion (from NEB), the manufacturer recommended to digest 1 µg of substrate protein with 50 ng of endoproteinase Glu-C at 37°C for 16h. The buffer reaction consisted in 50 mM Tris-HCI pH 8.0 and 0.5 mM GluC-GluC.

### Size exclusion chromatography (SEC)

Size-exclusion chromatography of purified and digested fusion protein was performed using an AKTA Pure system (GE Healthcare) in order to separate the protein of interest from the carrier.

A Superdex S30 Increase G10/300 GL column (GE Healthcare) and a HiLoad 26/600 Superdex 30 prep grade column were first calibrated using two standards diluted with 2X PBS buffer (or appropriate buffer for the further step): aprotinin (bovine lung; 6.5 kDa), and glycine (75 Da).

After a washing step in water, the Superdex S30 Increase G10/300 GL column was equilibrated in running buffer (2X PBS, pH 7.4, or 1X PBS, pH 7.4 or appropriate buffer for the further step) and 200 to 500 µL samples were run through the column at a rate of 0.5 mL/min. Elution of protein was detected by measuring optical absorbance at 280, 224 and 214 nm. 0.5 mL fractions were collected and analyzed by SDS-PAGE followed by Western-Blot or stained by Coomassie Blue dye.

After a washing step in water, the HiLoad 26/600 Superdex 30 prep grade column was equilibrated in running buffer (2X PBS, pH 7.4, or 1X PBS, pH 7.4 or appropriate buffer for the further step) and samples (4 to 30 mL) were run through the column at a rate of 2.6 mL/min. Elution of proteins was detected by measuring optical absorbance at 280, 224 and 214 nm. 4 mL fractions were collected and analyzed by SDS-PAGE followed by Western-Blot.

In some embodiment, the elution fractions of interest were pooled and evaporated using a SpeedVac (Eppendorf). The peptides or polypeptides or proteins present in these evaporated samples were subjected to Edman degradation to confirm the amino acids sequence at the N-terminus of the protein of interest.

### Example 2

### Production of aprotinin in the chloroplast of Chlamydomonas reinhardtii by chloroplast genome transformation

### Design of aprotinin

The nucleic acid and amino acids sequences of the mature *Bos Taurus* (bovine) aprotinin (SEQ. ID N°1 and 2) are known in the art and were extracted from UniProt (P00974) and the GenBank Accession Number X05274; (Creighton and Charles, 1987).

In *Chlamydomonas reinhardtii,* the codon usage has been shown to play a significant role in protein accumulation (Franklin *et al.,* 2002; Mayfield and Schultz, 2004).

The nucleic acid sequence encoding aprotinin alone or chimeric aprotinin or fused with recombinant peptide/polypeptide/proteins were modified in order to improve its expression in *C. reinhardtii* host cell.

Methods for altering nucleic acids for improved expression in host cell are known in the art, particularly in algae cell, particularly in *C. reinhardtii.*

A codon usage database is found at http://www.kazusa.or.jp/codon/. (See the codon usage table for chloroplast genome of *C. reinhardtii;* Figure 1).

For improving expression in *C. reinhardtii* chloroplast of the gene of interest of the present invention, codons from the native bovine aprotinin sequence which are not commonly used, were replaced with a codon coding for the same or a similar amino acid residue that is more commonly used in the *C. reinhardtii* chloroplast codon bias. In addition, other codons were replaced to avoid sequences of multiple or extended codon repeats, or some restriction enzyme site, or having a higher probability of secondary structure that could reduce or interfere with expression efficiency.

In order to check and to fulfill all criteria mentioned above, the nucleic acid sequence of aprotinin were optimized by the software GENEius of Eurofins and the appropriate codon usage for *C. reinhardtii* chloroplast genomes.

After optimization, the gene encoding aprotinin (APRO) were operationally fused at its 5'end to a codon optimized nucleic acid sequence encoding the HA Tag (HA) followed by a signal peptide, the 3XFlag Tag (3F) and the cleavage site recognized by the Factor Xa protease (FX). This fusion protein is named HA-SP-3F-FX-APRO or chimeric aprotinin (SEQ ID N°70 and corresponding nucleic acid sequence SEQ ID N°71).

The nucleic acid sequence encoding the Signal Peptide (SP; SEQ ID N°72: NNNDLFQASRRRFLAQLGGLTVAGMLGPSLLTPRRATAAQA and corresponding nucleic acid sequence SEQ ID N°73) is extracted from the sequence of the *E. coli TorA* gene encoding the Trimethylamine-N-oxide reductase 1 (UniProt number P33225).

The codon usage of the HA Tag, the signal peptide, the 3XFlag Tag and the recognition site of Factor Xa were previously optimized for the expression of another transgenes and as described previously using the codon usage for *C. reinhardtii* chloroplast genome but independently of the aprotinin optimization.

This fusion gene *ha-sp-3f-fx-apro* (SEQ ID N°71) was synthesized and cloned by Eurofins Genomics in the vector pEX-A2 resulting in vector pAL60.

### Construction of transformation vectors

Several chloroplast transformation vectors for the expression of different chimeric aprotinins and aprotinin were constructed (Figure 2).

The transformation vector pAU76 allowed the production of the chimeric aprotinin HA-SP-3F-FX-APRO (SEQ ID N°70): aprotinin was fused at its N-terminus to an amino acid sequence containing the HA epitope Tag (HA) followed by the signal peptide (SP), the 3XFlag epitope Tag (3F) and the cleavage site for Factor Xa protease (FX).

After the production in algae chloroplasts of the chimeric aprotinin HA-SP-3F-FX-APRO, the N-terminus fragment HA-SP will be cleaved during protein translocation into the thylakoids, and the following recombinant protein 3F-FX-APRO will be produced *in vivo.*

The protease cleavage site FX allows the *in vitro* release of aprotinin by adding Factor Xa protease to a sample of the chimeric aprotinin 3F-FX-APRO (SEQ ID N°74).

The codon usage of the nucleic sequence encoding the HA-SP followed by the 3XFlag epitope Tag (3F) and the cleavage site for the Factor Xa protease (FX) were optimized as described previously using the usage codon for *C. reinhardtii* chloroplast genome.

The nucleic acid sequence of the chimeric aprotinin was amplified by PCR from the vector pAL60 using the primers O5'AS_GibaproBE (SEQ ID N°75) and O3'AS_GibaproBE (SEQ ID N°76).

This PCR fragment of 267 pb containing the chimeric aprotinin was cloned between the *psbD* promoter/5'UTR and the *atpA* 3'UTR into the transformation vector pLE63 linearized by *BamH*I/*Pme*I digestions to give pAU76. Thus the expression cassette of the goi was *PpsbD-5'UTRpsbD-ha-sp-3f-fX-apro-3'UTRatpA* (SEQ ID N°77).

The chloroplast transformation vector pLE63 contained two expression cassettes (Figure 2) for the expression of the genes encoding the selectable marker (gos) and the recombinant protein of interest (*goi*). The selection cassette contained the selectable marker *aadA* gene coding aminoglycoside 3"-adenylyltransferase and conferring the resistance to spectinomycin and streptomycin. This gene was operationally linked at its 5' end to the *C. reinhardtii 16S rRNA* promoter (*Prrn*) fused to the *atpA* 5'UTR and at its 3'end to the 3'UTR of the *C. reinhardtii rbcL gene.* In the second cassette, stable expression of the recombinant *goi* was controlled by the promoter and 5'UTR from *C. reinhardtii psbD* and the 3'UTR from *C. reinhardtii atpA.* In this expression cassette, the *goi* was fused at its 5'end to a nucleic acid sequence *ha-sp-3F.*

These two expression cassettes are flanked by a left (LHRR) and right (RHRR) endogenous homologous recombination sequences which are identical to those surrounding the targeted integration site into the *C. reinhardtii* chloroplast genome. The choice of the insertion site within the chloroplast genome was generally made such as not to disrupt an essential gene or interrupt the expression of a polycistronic unit.

The chloroplast transformation vector pLE63 allowed the targeted integration of the transgenes into the chloroplast genome of *C. reinhardtii* between the *5S rDNA* and *psbA* genes (and derived from instance from GenBank Accession Number NC005352).

The chloroplast transformation vector pAU97 allowed the production of the chimeric aprotinin HA-SP-3F-APRO (SEQ ID N°7 and 8): aprotinin was fused at its N-terminus with the HA Tag followed by the signal peptide, and the 3XFlag Tag. In this case, the release of aprotinin can be performed *in vitro* by enterokinase which cleaved the protein sequence after the second lysine amino acid in the motif sequence DYKDDDDK (SEQ ID N°60). The gene GNC-SFAPRO encoding this fusion protein was synthetized by Eurofins and cloned directly using Gibson assembly reaction (NEB) into pLE63 linearized by *BamH*I/*Pme*I digestions to give pAU97

The chloroplast transformation vector pAU94 allowed the production of the chimeric aprotinin HA-SP-APRO-LG-FX-NY2-3F (SEQ ID N° 78 AND 79). The N-terminus of aprotinin is fused with the HA Tag followed by the signal peptide SP. Its C-terminus is linked to a linker LG followed by the Factor Xa cleavage site, an hexapeptide and the 3XFlag Tag. pAU94 was obtained by a Gibson assembly cloning into pLE63 linearized by *BamH*I/*Pme*I of a PCR fragment of 563 bp containing the nucleic sequence encoding the chimeric aprotinin HA-SP-APRO-LG-FX-NY2-3F.

In the case of algae chloroplasts transformed by pAU97 or pAU94, the N-terminus fragment HA-SP will be cleaved during the translocation of the protein HA-SP-3F-APRO or HA-SP-APRO-LG-FX-NY2-3F, respectively, during protein translocation into the thylakoids, and the following recombinant protein 3F-APRO (SEQ ID N°4) or APRO-LG-FX-NY2-3F (SEQ ID N°80) will be produced *in vivo.*

The chloroplast transformation vector pAU27 allowed the production of the chimeric aprotinin into the stroma of algae chloroplast 3F-APRO (SEQ ID N°4). It was obtained using Gibson assembly reaction for cloning into pAU63 linearized by *Nco*I/*Pme*I the gene GNC-FAPRO synthetized by Eurofins.

### Transformation of algae

The transformation vectors pAU76, pAU97, pAU94 and pAU27 were bombarded in *Chlamydomonas* cell (137c and CW15) as described in the Example 1.

In order to identify stable integration of the recombinant genes encoding chimeric aprotinin into the chloroplast algal genome, spectinomycin resistant colonies were screened by PCR amplification of the gene of interest using the primers O5'ASTatpA (SEQ ID N°81) or O5'ASTatpA2 (SEQ ID N°82) and O3'SUTRpsbD (SEQ ID N°83) annealing, respectively, in the *atpA* 3'UTR and in *psbD* promoter.

### Analyses and results

Western Blot analysis were performed on total soluble protein samples extracted from several transformants obtained after transformation with pAU76, pAU97, pAU94 or pAU27 (Figures 3, 4 and 5).

The results show that all chimeric aprotinin were produced, being probed by the anti-Flag M2 antibody.

Moreover, in the case of transformants AU76, AU97 and AU94, the HA Tag and the signal peptide seems to be cleaved because Western Blot analyses performed on total soluble protein extracts show no specific protein recognized by the anti-HA antidody (Figure 7). Thus, the three types of fusion protein produced *in vivo* in the transformants AU76, AU97 and AU94 are 3F-FX-APRO, 3F-APRO and APRO-LG-FX-NY2-3F, respectively.

### Confirmation of the chimeric aprotinin activity using the trypsin binding assay

Aprotinin is a 58 amino acids monomeric polypeptide with a three-dimensional conformation maintained by three disulfide bridges.

Aprotinin, also known as a pancreatic trypsin inhibitor, binds with high affinity and specificity to serine proteases as for instance trypsin. The formed BPTI-trypsin complex is extremely stable.

25 µg of total soluble protein or purified protein of interest from transformants were incubated separately for 30 minutes at 37°C with 1.8 µg of trypsin (Sigma). As a positive control, 100 ng of standard aprotinin mixed with 50 µg of total soluble protein from WT strain were incubated in parallel and in the same conditions. Afterwards, samples were separated by SDS-PAGE under non-reducing conditions.

Western blot analysis performed using monoclonal antibodies directed against aprotinin showed for standard aprotinin and chimeric aprotinin from the transformant CW-AU76-1, the apparition of a higher molecular weight band as soon as trypsin was added to the samples (Figure 6). This new band corresponded to the specific complex of aprotinin and chimeric aprotinin bounded to trypsin.

This experiment demonstrates that the totality of chimeric aprotinin produced in chloroplast transformants with vectors pAU76 has an active conformation able to bind to trypsin. This also strongly suggests that the three disulfide bonds which maintain the structure of aprotinin are formed and correctly paired.

Chimeric aprotinin extract from the transformant CW-AU76-1 were purified by affinity chromatography using an anti-FLAG M2 affinity resin (Figure 7).

### Example 3

### Production of aprotinin as a fusion partner for the production of a polypeptide of elastin in the chloroplast of Chlamydomonas reinhardtii bv chloroplast genome transformation

### Construction of transformation vectors (pLA01, pLA02, pLA03 and pLA04)

In chloroplast transformation vector, ELP4, an elastin like polypeptide consisted of a repeat of the VGVAPG hexapeptide (SEQ ID N°34), more particularly of a 4-fold repeat of this hexapeptide (SEQ ID N°39: VGVAPGVGVAPGVGVAPGVGVAPG), was expressed in a fusion protein in which it was fused, at the C-terminus of the chimeric aprotinin HA-SP-3F-FX-APRO. This fusion partner contained aprotinin fused at its N-terminus to an amino acid sequence made of the HA epitope Tag (HA) followed by the signal peptide (SP), the 3xFlag epitope Tag (3F), and the cleavage site for Factor Xa (FX; SEQ ID N°102: IEGR). The Flag epitope Tag sequence (SEQ ID N°60: DYKDDDDK) which is the cleavage site for the enterokinase was inserted between the chimeric aprotinin and ELP4 in order to allow the release of ELP4 from aprotinin by *in vitro* site specific proteolysis of the fusion protein with enterokinase.

The nucleic acid sequence encoding ELP4 was first codon-optimized using the same method described in Example 1 and the codon usage for chloroplast genome of *Chlamydomonas.* The resulting sequence was used to design two overlapping oligomers O5'Gibs-ELP4 (SEQ ID N°84) and O3'Gibs-ELP4 (SEQ ID N°85) which were used as primers and template to amplify by PCR the fragment FGibs-ELP4 of 194 bp. This amplified DNA were cloned using the Gibson Assembly Master Mix from New England Biolabs (as recommended by the manufacturer) into the chloroplast transformation vector pAU76 described in the Example 2 and linearized by *Pme*I to form the vector pLA00.

The nucleic acid sequence encoding ELP4 were amplified by PCR from pLA00 using the primers O5'Gibs501BE (SEQ ID N°86) and O3'Gibs501BE (SEQ ID N°87). The PCR fragment FPCR-AP-FELP4 (SEQ N°125) of 359 pb were cloned using the Gibson Assembly Master Mix into the pAL863 linearized by *BamH*I and *Pme*I to form the vector pLA01. The transformation vector pLA01 allowed the production of the fusion protein HA-SP-3F-FX-APRO-F-ELP4 (SEQ ID N°88) containing ELP4 linked at its N-terminus to the chimeric aprotinin HA-SP-3F-FX-APRO followed by the 1X Flag Tag (Figure 8).

The transformation vector pLA02 was obtained by cloning by Gibson Assembly into pLE63 (linearized by *BamH*I and *Pme*I), the PCR fragment FPCR-FELP4-HA (SEQ ID N°89) (359 pb) amplified from pLA00 with primers O5'Gibs02BE (SEQ N°90) and O3'Gibs02BE (SEQ N°91). The transformation vector pLA02 allowed the production of fusion protein HA-SP-3F-1F-ELP4 containing ELP4 linked at its N-terminus to the chimeric sequence HA-SP-3F followed by the 1X Flag Tag (Figure 8).

In the case of algae chloroplasts transformed by pLA01 or pLA02 and if the signal peptide SP is cleaved after translocation of the fusion protein into the lumen of thylakoids, two other different proteins can be produced *in vivo,* 3F-FX-APRO-1F-ELP4 or 3F-1F-ELP4 (SEQ ID N°92).

In both types of transformation vectors, the release of ELP4 from the fusion proteins can be performed *in vitro* by enterokinase digestion which cleaved the protein sequence after the second lysine amino acid in the motif sequence DYKDDDDK present in the 1X Flag Tag just upstream ELP4.

The elastin like polypeptide named ELPE4 consisted of a repeat of the VGVAPGE (SEQ ID N°38), a derivative of the peptide VGVAPG, more particularly of a 4-fold repeat of this peptide (SEQ N°40, VGVAPGEVGVAPGEVGVAPGEVGVAPGE). In chloroplast transformation vector, ELPE4 was also expressed in a fusion protein in which it was fused at the C-terminus of the chimeric aprotinin HA-SP-3F-FX-APRO (SEQ ID N°5) (Figure 8).

In order to separate *in vitro* by the ELPE4 from the carrier, a flexible linker LGM (SEQ ID N°65: RSGGGGSSGGGGGGSSRS) followed by a cleavage site for TEV protease (TV; SEQ ID N°93: ENLYFQG) or enterokinase (EK; SEQ ID N°94: DDDDK) were added.

Two types of fusion proteins have been produced from two different chloroplast expression vectors: HA-SP-3F-FX-APRO-LGM-TV-ELPE4 or HA-SP-3F-FX-APRO-LGM-EK-ELPE4

The nucleic acid sequence encoding LGM-TV-ELPE4 or LGM-EK-ELPE4 were codon-optimized using also the same method described in Example 1 and the codon usage for chloroplast genome of *C. reinhardtii.* After codon optimization, the different synthetic genes *Igm-tv-elpe4* (SEQ ID N°95) and *Igm-ek-elpe4* (SEQ ID N°96) were synthetized by Eurofins. These optimized genes were cloned by Gibson assembly downstream the gene encoding the carrier into an expression cassette (SEQ N°97) present in the chloroplast transformation vector pAU76 linearized by *Pme*I to give respectively, pLA03 and pLA04 (Figure 8).

### Transformation of algae

The transformation vectors pAL01, pLA02, pLA03 and pLA04 were bombarded in *C. reinhardtii* cell (137c and CW15) as described in the Example 1.

In order to identify stable integration of the recombinant genes encoding fusion protein into the chloroplast algal genome, spectinomycin resistant colonies were screened by PCR analysis using the primers O5'ASTatpA2 (SEQ N°82) and O3'SUTRpsbD (SEQ N°83) annealing, respectively, in the *atpA* 3'UTR and *psbD* 5'UTR.

### Analyses and results

Western Blot analysis performed using anti-Flag antibody on total soluble proteins extracted from different independent strains of LA01, LA03 or LA04 transformants revealed that the fusion proteins HA-SP-3F-FX-APRO-F-ELP4 (SEQ ID N°88), HA-SP-3F-FX-APRO-LGM-TV-ELPE4 (SEQ ID N°100) and HA-SP-3F-FX-APRO-LGM-EK-ELPE4 (SEQ ID N°101) were produced in the *C. reinhardtii* chloroplast.

As shown in the Figure 9, Western Blot analysis showed that the ELP4 polypeptide fused to 3F-FX-APRO is very well produced in the LA01 transformants using anti-Flag antibody. Moreover, in all LA01 transformants, the HA epitope Tag and the signal peptide seems to be cleaved because Western blots performed on the same total soluble protein extracts showed that the primary anti-HA antibody didn't recognize any fusion protein (Figure 9).

In the LA02 transformants, no recombinant protein were detected (Figure 9) demonstrating that the fusion of ELP4 to 3F-FX-APRO (or aprotinin as a general manner) allows the accumulation of ELP4.

Biomass of one transformant CW-LA01 was produced. Cell pellet was resuspended in sonication buffer.

Fusion protein were purified by anti-Flag M2 affinity chromatography. Elution fraction containing the fusion protein were identify by Western Blot analysis, dialyzed and concentrated. Enterokinase protease digestions were performed followed by a size exclusion chromatography (HiLoad 26/00 Superdex 30) allowing the purification of the polypeptide ELP4.

The same method was applied for the purification of the ELPE4. The fusion protein were purified by affinity chromatography. Elution fraction containing the fusion protein were identify by Western Blot analysis, dialyzed and concentrated. Enterokinase or TEV protease digestions were performed depending on the transformant followed by a size exclusion chromatography (HiLoad 26/00 Superdex 30) allowing the purification of the polypeptide ELPE4.

In order to cleave by endoproteinase the polypeptides ELPE4 into peptides VGVAPGE, the SEC elution fractions were evaporated and dialyzed for salts removing and buffer changing, using a dialysis tube with a 1 kDa cutoff.

After digestion by the Glu-C_endoproteinase of the dialyzed samples as described in the Example 1, the released peptides were purified by a size exclusion chromatography.

### Example 4

### Production of aprotinin as a fusion partner for the production of a mono and polypeptides of KTTKS and derivatives in the chloroplast of Chlamydomonas reinhardtii by chloroplast genome transformation

### Construction of transformation vectors (pNY18, pNY19, pNY13, pNY14, pNY15, pNY16)

Several chloroplast transformation vectors were constructed in order to express the peptides KTTKS (named NY2 of SEQ ID N°18) and GKTTKS (named GNY2 of SEQ ID N°19) and polypeptides of KTTKS derivatives in a fusion protein using aprotinin as a carrier (Figure 10).

In chloroplast transformation vector, the peptide NY2 and the polypeptides (NY3a)x5 (KTTKSDKTTKSDKTTKSDKTTKSDKTTKSD) (SEQ ID N°26) and (NY3b)x5 (KTTKSEKTTKSEKTTKSEKTTKSEKTTKSE) (SEQ ID N°28) were produced in fusion proteins in which they were fusedat the C-terminus of the chimeric aprotinin HA-SP-3F-FX-APRO (SEQ ID N°5). This fusion partner contained aprotinin fused at their N-terminus to an amino acid sequence made of the HA epitope Tag (HA) followed by the signal peptide (SP), the 3XFlag epitope Tag (3F), and the cleavage site for Factor Xa (FX; IEGR (SEQ ID N°102)).

In the fusion protein, the peptide NY2 or the polypeptides (NY3a)x5 and (NY3b)x5 were separated from the carrier by the flexible linker LGM (SEQ ID N°65: RSGGGGSSGGGGGGSSRS) followed by a cleavage site for TEV protease (TV; SEQ ID N°93; ENLYFQG) or enterokinase (EK; SEQ ID N°94; DDDDK).

Therefore, different fusion proteins were produced in independent algae transformants, as for instance, the protein called HA-SP-3F-FX-APRO-LGM-EK-NY2 (SEQ ID N°103 and 104), HA-SP-3F-FX-APRO-LGM-TV-NY2 (SEQ ID N°105 and 106), HA-SP-3F-FX-APRO-LGM-EK-(NY3a)x5 (SEQ ID N°107 and 108), HA-SP-3F-FX-APRO-LGM-TV-(NY3a)x5 (SEQ ID N°109 and 110), HA-SP-3F-FX-APRO-LGM-EK-(NY3b)x5 (SEQ ID N°111 and 112), HA-SP-3F-FX-APRO-LGM-TV-(NY3b)x5 (SEQ ID N°113 and 114) (Figure 10).

After their production in algae chloroplasts, the signal peptide (SP) targeted these fusion proteins into the thylakoids. During protein translocation, the N-terminus fragment HA-SP were cleaved and the following other recombinant proteins were produced *in vivo,* 3F-FX-APRO-LGM-TV-(NY3b)x5 (SEQ ID N°115), 3F-FX-APRO-LGM-EK-(NY3b)x5 (SEQ ID N°116), 3F-FX-APRO-LGM-TV-(NY3a)x5 (SEQ ID N°117), 3F-FX-APRO-LGM-EK-(NY3a)x5 (SEQ ID N°118), 3F-FX-APRO-LGM-EK-NY2 (SEQ ID N°119) and 3F-FX-APRO-LGM-TV-NY2 (SEQ ID N°120).

The release of the peptides and the polypeptides from the chimeric aprotinin has been performed *in vitro* by site specific proteolysis of the fusion protein with enterokinase or TEV proteases.

After the cleavage of the fusion protein HA-SP-3F-FX-APRO-LGM-TV-NY2 or 3F-FX-APRO-LGM-TV-NY2 by the TEV protease, the released peptide was GNY2 (GKTTKS). In the case of the TEV digestion of the fusion proteins HA-SP-3F-FX-APRO-LGM-TV-(NY3a)x5 (or 3F-FX-APRO-LGM-TV-(NY3a)x5) and HA-SP-3F-FX-APRO-LGM-TV-(NY3b)x5 (or 3F-FX-APRO-LGM-TV-(NY3b)x5), the released polypeptides was G((NY3a)x5) and G((NY3b)x5), respectively.

In *Chlamydomonas reinhardtii,* the codon usage in the nucleic acid sequence encoding protein of interest has been shown to play a significant role in protein accumulation (Franklin *et al.,* 2002; Mayfield and Schultz, 2004).

The nucleic acid sequence encoding the chimeric aprotinin were designed and optimized in order to improve their expression in *C. reinhardtii* host cells

Methods for altering polynucleotides for improved expression in host cell are known in the art, particularly in algae cell, particularly in *C. reinhardtii.*

A codon usage database is found at http://www.kazusa.or.jp/codon/. (See the codon usage for chloroplast genome of *C. reinhardtii;* Figure 1).

For improving expression in *C. reinhardtii* chloroplast of the gene of interest, codons from their native sequence which are not commonly used, were replaced with a codon coding for the same or a similar amino acid residue that is more commonly used in the *C. reinhardtii* chloroplast codon bias. In addition, other codons were replaced to avoid sequences of multiple or extended codon repeats, or some restriction enzyme site, or having a higher probability of secondary structure that could reduce or interfere with expression efficiency.

In order to check and to fulfill all criteria mentioned above, the amino acid sequence of the protein of interest were also optimized by the software GENEius of Eurofins using the appropriate usage codon for *C. reinhardtii* chloroplast.

After optimization, the gene encoding aprotinin (APRO) were operationally fused at its 5'end to a codon optimized nucleic acid sequence encoding the HA epitope Tag (HA) followed by a signal peptide, the 3XFlag epitope Tag (3F) and the cleavage site recognized by the Factor Xa protease (FX) to form the chimeric aprotinin HA-SP-3F-FX-APRO (SEQ ID N°5 AND 6).

The nucleic acid sequence encoding the recombinant peptide of KTTKS or GKTTKS, or polypeptide of KTTKS or their derivatives were designed and optimized as mentioned above in order to improve their expression in *C. reinhardtii* host cells.

After codon optimization, the different synthetic genes GNC-LENY3a2 (SEQ ID N°121), GNC-LENY3b1 (SEQ ID N°122), GNC-LTNY3a2 (SEQ ID N°123) and GNC-LTNY3b1 (SEQ ID N°124) encoding respectively the polypeptides (NY3a)x5, (NY3b)x5), G((NY3a)x5), G((NY3b)x5) were synthetized by Eurofins. These optimized genes were cloned by Gibson assembly downstream the gene encoding the carrier into an expression cassette present in the chloroplast transformation vector pAU76 linearized by *Pme*I to give respectively, pNY16, pNY14, pNY15, and pNY13.

The chloroplast transformation vectors pNY13 and pNY14 allowed the expression of the polypeptide (NY3b)x5 in the fusion proteins HA-SP-3F-FX-APRO-LGM-TV-(NY3b)x5 and HA-SP-3F-FX-APRO-LGM-EK-(NY3b)x5, respectively (Figure 10).

The chloroplast transformation vectors pNY15 and pNY16 allowed the expression of the polypeptide (NY3a)x5 in the fusion proteins HA-SP-3F-FX-APRO-LGM-TV-(NY3a)x5 and HA-SP-3F-FX-APRO-LGM-EK-(NY3a)x5, respectively (Figure 10).

The optimized genes GNC-ALENY2 (SEQ ID N°99) and GNC-ALTNY2 (SEQ ID N°98) were cloned by Gibson assembly into an expression cassette present in the chloroplast transformation vector pAU63 linearized by *BamH*I and *Pme*I digestions to give respectively, pNY18 and pNY19.

The chloroplast transformation vectors pNY18 and pNY19 allowed the expression of the peptide NY2 in the fusion proteins HA-SP-3F-FX-APRO-LGM-EK-NY2 and HA-SP-3F-FX-APRO-LGM-TV-NY2, respectively (Figure 10).

The expression vectors pAU76 and pAU63 for chloroplast genome transformation contained two expression cassettes (Figure 10) for the expression of the genes encoding the selectable marker and the fusion protein.

These two expression cassettes are flanked by a left (LHRR) and right (RHRR) endogenous homologous recombination sequences which are identical to those surrounding the targeted integration site into the *C. reinhardtii* chloroplast genome. The chloroplast transformation vectors i allow the targeted integration of the transgenes into the chloroplast genome of *C. reinhardtii* between the *5S rDNA* and *psbA* genes (and derives from instance from GenBank Accession Number NC005352).

The selectable marker gene was the *aadA* gene coding aminoglycoside 3"-adenylyltransferase and conferring the resistance to spectinomycin and streptomycin. The gene is operationally linked at its 5' end to the *C. reinhardtii 16S rRNA* promoter (*Prrn*) fused to the *atpA* 5'UTR (SEQ ID N°67) and at its 3' end to the 3'UTR of the *C. reinhardtii rbcL gene* (SEQ ID N°69) (Figure 10).

Stable expression and translation of the fusion protein gene is controlled by the promoter and 5'UTR from the *C. reinhardtii psbD* (SEQ ID N°66) and the 3'UTR from *C. reinhardtii atpA* (SEQ ID N°67) (Figure 10).

The construction of pAU94 was previously described in the Example 2. The chloroplast transformation vector pAU94 allowed the production of the chimeric aprotinin HA-SP-APRO-LG-FX-NY2-3F (SEQ ID N° 78 and 79).

### Transformation of algae

The transformation vectors pNY18, pNY19, pNY13, pNY14, pNY15, pNY16 were bombarded in *C. reinhardtii* cell (137c and CW15) as described in the Example 1. In order to identify stable integration of the recombinant genes encoding fusion protein into the chloroplast algal genome, spectinomycin resistant colonies were screened by PCR analysis. For positive PCR screens of the fusion protein gene, the primers

O5'ASTatpA2 5'-CCTACTTAATTAAAAACtgcagtagctagctctgc-3' (SEQ ID N°82) and O3'SUTRpsbD 5'-cgatgagttgtttttttattttggagatacacgc-3' (SEQ ID N°83) annealing, respectively, in the *atpA* 3'UTR and *psbD* 5'UTR were used.

### Analyses and results

Western Blot analysis of total soluble proteins extracted from different independent strains transformed with different expression vectors revealed that the fusion proteins HA-SP-3F-FX-APRO-LGM-EK-NY2, HA-SP-3F-FX-APRO-LGM-TV-NY2, HA-SP-3F-FX-APRO-LGM-EK-(NY3a)x5, HA-SP-3F-FX-APRO-LGM-TV-(NY3a)x5, HA-SP-3F-FX-APRO-LGM-EK-(NY3b)x5, HA-SP-3F-FX-APRO-LGM-TV-(NY3b)x5 and HA-SP-APRO-LG-FX-NY2-3F were produced significantly (Figure 5, 11 and 12).

Moreover, for all transformants, the HA Tag and the signal peptide SP seems to be cleaved because Western Blot analyses performed on total soluble protein extracts show that no specific protein is recognized by the anti-HA antibody.

The comparison of the fusion protein amounts between the different types of transformants, performed by Western blot analyses, seems to show that the clones CW-AL813-4 and CW-AL818-6 would strongly produce HA-SP-3F-FX-APRO-LGM-TV-(NY3b)x5 (0.1% of total soluble proteins; TSP) and HA-SP-3F-FX-APRO-LGM-EK-NY2 (0.089% TSP), respectively.

### Purification of peptides and polypeptides of KTTKS or derivatives

About 80 g of cells for each transformants CW-NY13-4 and CW-NY18-6 were produced from several 1L cascade cultures. Algae cells were resuspended and sonicated as described in the Material and Methods. 850 mL of total soluble protein extract for each transformants CW-NY13-4 and CW-NY18-6 were obtained.

In order to concentrate these extract volumes of 850 mL, a supplementary steps of ammonium sulfate precipitation and dialysis were added before the affinity chromatography as described in Example 1.

Fusion protein were purified by anti-Flag M2 affinity chromatography. Elution fractions were analysed by Western Blot analysis. The results, shown in Figure 13, revealed the effectiveness of the affinity chromatography purification of the fusion proteins produced in the transformants CW-NY13-4 and CW-NY18-6.

The elution fraction from affinity chromatography containing the fusion protein 3F-FX-APRO-LGM-EK-NY2 or 3F-FX-APRO-LGM-TV-(NY3b)x5 were dialyzed in dialysis cassettes (3.5 kDa MWCO) against the buffer used in the next step of protease digestion.and concentrated using centrifugal concentrators (3 kDa MWCO).

Then the cleavage of the peptide or polypeptide from the carrier was performed with a site specific proteolysis of the fusion protein APRO-LGM-EK-NY2 or APRO-LGM-TV-(NY3b)x5 using enterokinase or TEV protease, respectively.

After an overnight incubation at 4°C, the digestions of each fusion protein were injected on a HiLoad 26/600 Superdex 30 prep grade column and run at a rate of 2.6 mL/min. These size exclusion chromatography (SEC) experiments allowed the purification of the peptide NY2 and polypeptide (NY3b)x5.

Further analysis on the SEC purified polypeptide (NY3b)x5 and peptide NY2 were performed by high performance liquid chromatography (HPLC) on C18 and C4 large pore reverse phase columns with 215 nm UV detection and mass spectrometry.

In order to cleave the polypeptides (NY3b)x5 into peptides by endoproteinase, the SEC elution fractions were evaporated and dialyzed for salts removing and buffer changing, using a dialysis tube with a 1 kDa cutoff.

After digestion by the Glu-C_endoproteinase of the dialyzed samples as described in the Example 1, the released peptides were purified by a size exclusion chromatography.

## Claims

1. Use of aprotinin as a carrier to produce a recombinant protein, polypeptide or peptide in algae, wherein aprotinin and said recombinant protein, polypeptide or peptide are fused together to form a fusion protein.

2. A method to produce a recombinant protein, polypeptide or peptide in algae, wherein said method comprises transforming transformation of algae with a nucleic acid sequence encoding a fusion protein, wherein said fusion protein comprises aprotinin and said recombinant protein, polypeptide or peptide.

3. Method according to claim 2, comprising the following steps:
(i) providing a nucleic acid sequence encoding a fusion protein, wherein said fusion protein comprises aprotinin and said recombinant protein, polypeptide or peptide;
(ii) introducing the nucleic acids sequence according to (i) into an expression vector which is capable of expressing the nucleic acid sequence in an algae host cell; and
(iii) transforming the genome of algae host cell by the expression vector.

4. Method according to claim 3, further comprising:
(iv) identifying the transformed algae host cell;
(v) characterizing the microalgae host cell for the production of recombinant fusion protein;
(vi) extracting the recombinant fusion protein; and optionally
(vii) purifying the fusion protein.

5. A recombinant algae comprising a nucleic acid sequence encoding a fusion protein, wherein said fusion protein comprises aprotinin and a recombinant protein, polypeptide or peptide.

6. Use according to claim 1 or method according to any of claims 2 to 4 or recombinant algae according to claim 5, to increase accumulation and/or stability and/or solubility and/or folding and/or activity of recombinant proteins peptide, polypeptide or protein in algae, in particular in microalgae, more particularly in the chloroplast of microalgae.

7. Use according to any of claims 1 and 6 or method according to any of claims 2 to 4 and 6 or recombinant algae according to claim 5, wherein said protein, polypeptide or peptide is chosen from, collagen, collagen like and matricins proteins, polypeptides or peptides

8. Use or method or recombinant algae according to claim 7, wherein said matricins proteins, polypeptides or peptides, are chosen from elastin and elastin like proteins.

9. Use according to any of claims 1 and 6 to 8 or method according to any one of claims 2 to 4 and 6 to 8 or recombinant algae according to any of claims 5 to 8, wherein said algae is chosen from the group consisting of *Chlorophyta, Chlorophyceae, Pleurastrophyceae, Prasinophyceae, Chromophyta, Bacillariophyceae, Chrysophyceae, Phaeophyceae, Eustigmatophyceae, Haptophyceae, Raphidophyceae, Xanthophyceae, Cryptophyta, Cryptophyceae, Rhodophyta, Porphyridiophycea, Stramenopiles, Glaucophyta, Glaucocystophyceae, Chlorarachniophyceae, Haptophyceae, Dinophyceae, Scenedesmaceae, Euglenophyta, Euglenophyceae* and *Cyanophyceae.*

10. Use or method or recombinant algae according to claim 9, wherein said algae is chosen from the group consisting of *Chlamydomonas, Chlorella, Dunaliella, Haematococcus, diatoms, Scenedesmaceae, Tetraselmis, Ostreococcus, Porphyridium, Nannochloropsis, Arthrospira platensis, Arthrospira maxima, Anabaena* sp. PCC7120, Leptolyngbya sp, *Synechocystis sp,* and *Synechococcus sp.*

11. Use according to any of claims 1 and 6-10 or method according to any of claims 2 to 4 and 6 to 10 or recombinant algae according to any of claims 5 to 10, wherein said recombinant protein, polypeptide or peptide is fused to the C-terminus of aprotinin.

12. Use according to any of claims 1 and 6-11 or method according to any of claims 2 to 4 and 6-11 or recombinant algae according to any of claims 5 to 11, wherein said fusion protein also comprises an epitope tag.

13. Use according to any of claims 1 and 6-12 or method according to any of claims 2 to 4 and 6-12 or recombinant algae according to any of claims 5 to 12, wherein said fusion protein also comprises a signal peptide.

14. Use according to any of claims 1 and 6-13 or method according to any of claims 2 to 4 and 6-13 or recombinant algae according to any of claims 5 to 13, wherein said fusion protein also comprises a protease specific cleavage site between the aprotinin and the recombinant protein, polypeptide or peptide.

15. Use of a recombinant algae according to any of claims 5-14, for producing a fusion protein, wherein said fusion protein comprises aprotinin and a recombinant protein, polypeptide or peptide.


SEQUENCE LISTING

<110>  Alganelle

<120>  Use of aprotinin as a carrier to produce a recombinant protein,
       polypeptide or peptide in algae

<130>  BET19L3921

<160>  125

<170>  PatentIn version 3.5

<210>  1
<211>  58
<212>  PRT
<213>  Bos taurus

<400>  1

<210>  2
<211>  174
<212>  DNA
<213>  Bos taurus

<400>  2
cggcctgact tctgcctaga gcctccatat acgggtccct gcaaggccag aattatcaga      60

tacttctaca acgccaaggc tgggctctgc cagacctttg tatatggcgg ctgcagagct     120

aaaagaaaca atttcaagag cgcagaggac tgcatgagga cctgtggtgg tgct           174

<210>  3
<211>  67
<212>  PRT
<213>  Artificial Sequence

<220>
<223>  HA-APRO

<400>  3

Tyr Pro Tyr Asp Val Pro Asp Tyr Ala Arg Pro Asp Phe Cys Leu Glu
1               5                   10                  15

Pro Pro Tyr Thr Gly Pro Cys Lys Ala Arg Ile Ile Arg Tyr Phe Tyr
            20                  25                  30

Asn Ala Lys Ala Gly Leu Cys Gln Thr Phe Val Tyr Gly Gly Cys Arg
        35                  40                  45

Ala Lys Arg Asn Asn Phe Lys Ser Ala Glu Asp Cys Met Arg Thr Cys
    50                  55                  60

Gly Gly Ala
65

<210>  4
<211>  82
<212>  PRT
<213>  Artificial Sequence

<220>
<223>  3F-APRO

<400>  4

Asp Tyr Lys Asp Asp Asp Asp Lys Asp Tyr Lys Asp Asp Asp Asp Lys
1               5                   10                  15

Asp Tyr Lys Asp Asp Asp Asp Lys Arg Pro Asp Phe Cys Leu Glu Pro
            20                  25                  30

Pro Tyr Thr Gly Pro Cys Lys Ala Arg Ile Ile Arg Tyr Phe Tyr Asn
        35                  40                  45

Ala Lys Ala Gly Leu Cys Gln Thr Phe Val Tyr Gly Gly Cys Arg Ala
    50                  55                  60

Lys Arg Asn Asn Phe Lys Ser Ala Glu Asp Cys Met Arg Thr Cys Gly
65                  70                  75                  80

Gly Ala

<210>  5
<211>  140
<212>  PRT
<213>  Artificial Sequence

<220>
<223>  HA-SP-3F-FX-APRO

<400>  5

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala
    130                 135                 140

<210>  6
<211>  423
<212>  DNA
<213>  Artificial Sequence

<220>
<223>  nucleic sequence encoding HA-SP-3F-FX-APRO

<400>  6
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaagg atccattgaa     240

ggtcgtagac cagacttttg cttagaacca ccatatacag gtccatgtaa agctcgtatc     300

attcgctatt tctacaatgc aaaagcagga ttatgtcaaa catttgtgta tggaggttgt     360

cgtgctaaac gtaacaactt taaatcagct gaagattgta tgcgtacttg tggtggtgct     420

taa                                                                   423

<210>  7
<211>  134
<212>  PRT
<213>  Artificial Sequence

<220>
<223>  HA-SP-3F-APRO

<400>  7

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Arg Pro Asp Phe
65                  70                  75                  80

Cys Leu Glu Pro Pro Tyr Thr Gly Pro Cys Lys Ala Arg Ile Ile Arg
                85                  90                  95

Tyr Phe Tyr Asn Ala Lys Ala Gly Leu Cys Gln Thr Phe Val Tyr Gly
            100                 105                 110

Gly Cys Arg Ala Lys Arg Asn Asn Phe Lys Ser Ala Glu Asp Cys Met
        115                 120                 125

Arg Thr Cys Gly Gly Ala
    130


<210>  8
<211>  405
<212>  DNA
<213>  Artificial Sequence

<220>
<223>  nucleic sequence encoding HA-SP-3F-APRO

<400>  8
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaaag accagacttt     240

tgcttagaac caccatatac aggtccatgt aaagctcgta tcattcgcta tttctacaat     300

gcaaaagcag gattatgtca aacatttgtg tatggaggtt gtcgtgctaa acgtaacaac     360

tttaaatcag ctgaagattg tatgcgtact tgtggtggtg cttaa 405

<210> 9
<211> 108
<212> PRT
<213> Artificial Sequence

<220>
<223> HA-SP-APRO

<400> 9

Tyr Pro Tyr Asp Val Pro Asp Tyr Ala Asn Asn Asn Asp Leu Phe Gln
1               5                   10                  15

Ala Ser Arg Arg Arg Phe Leu Ala Gln Leu Gly Gly Leu Thr Val Ala
            20                  25                  30

Gly Met Leu Gly Pro Ser Leu Leu Thr Pro Arg Arg Ala Thr Ala Ala
        35                  40                  45

Gln Ala Arg Pro Asp Phe Cys Leu Glu Pro Pro Tyr Thr Gly Pro Cys
    50                  55                  60

Lys Ala Arg Ile Ile Arg Tyr Phe Tyr Asn Ala Lys Ala Gly Leu Cys
65                  70                  75                  80

Gln Thr Phe Val Tyr Gly Gly Cys Arg Ala Lys Arg Asn Asn Phe Lys
                85                  90                  95

Ser Ala Glu Asp Cys Met Arg Thr Cys Gly Gly Ala
            100                 105

<210> 10
<211> 324
<212> DNA
<213> Artificial Sequence

<220>
<223> nucleic sequence encoding HA-SP-APRO

<400> 10
tatccttatg atgtaccaga ttatgccaat aataacgact tatttcaagc atcacgtcgt 60

cgtttcttag ctcaattagg aggtttaaca gttgctggta tgttaggtcc atctttactt 120

actccacgta gagctacagc agctcaagct agaccagact tttgcttaga accaccatat 180

acaggtccat gtaaagctcg tatcattcgc tatttctaca atgcaaaagc aggattatgt 240

caaacatttg tgtatggagg ttgtcgtgct aaacgtaaca actttaaatc agctgaagat 300

tgtatgcgta cttgtggtgg tgct 324

<210>  11
<211>  123
<212>  DNA
<213>  Artificial Sequence

<220>
<223>  nucleic sequence encoding SP

<400>  11
aataataacg acttatttca agcatcacgt cgtcgtttct tagctcaatt aggaggttta      60

acagttgctg gtatgttagg tccatcttta cttactccac gtagagctac agcagctcaa      120

gct                                                                    123


<210>  12
<211>  41
<212>  PRT
<213>  Artificial Sequence

<220>
<223>  SP

<400>  12

Asn Asn Asn Asp Leu Phe Gln Ala Ser Arg Arg Arg Phe Leu Ala Gln
1               5                   10                  15


Leu Gly Gly Leu Thr Val Ala Gly Met Leu Gly Pro Ser Leu Leu Thr
            20                  25                  30


Pro Arg Arg Ala Thr Ala Ala Gln Ala
        35                  40


<210>  13
<211>  6
<212>  PRT
<213>  Artificial Sequence

<220>
<223>  cleavage site at the C-terminus of the SP

<400>  13

Ala Thr Ala Ala Gln Ala
1               5


<210>  14
<211>  91
<212>  PRT
<213>  Artificial Sequence

<220>
<223>  3F-FX-APRO

<400>  14

Ala Gln Ala Asp Tyr Lys Asp Asp Asp Asp Lys Asp Tyr Lys Asp Asp

1                   5                   10                  15

Asp Asp Lys Asp Tyr Lys Asp Asp Asp Asp Lys Gly Ser Ile Glu Gly
            20                  25                  30

Arg Arg Pro Asp Phe Cys Leu Glu Pro Pro Tyr Thr Gly Pro Cys Lys
        35                  40                  45

Ala Arg Ile Ile Arg Tyr Phe Tyr Asn Ala Lys Ala Gly Leu Cys Gln
    50                  55                  60

Thr Phe Val Tyr Gly Gly Cys Arg Ala Lys Arg Asn Asn Phe Lys Ser
65                  70                  75                  80

Ala Glu Asp Cys Met Arg Thr Cys Gly Gly Ala
                85                  90

<210>  15
<211>  6
<212>  PRT
<213>  Artificial Sequence

<220>
<223>  pal-KTTKS-OH

<220>
<221>  MISC_FEATURE
<222>  (1)..(1)
<223>  X=Palmitoyl

<400>  15

Xaa Lys Thr Thr Lys Ser
1               5

<210>  16
<211>  7
<212>  PRT
<213>  Artificial Sequence

<220>
<223>  Palmitoyl Hexapeptide-15

<220>
<221>  MISC_FEATURE
<222>  (1)..(1)
<223>  X=Palmitoyl

<400>  16

Xaa Gly Lys Thr Thr Lys Ser
1               5

<210>  17
<211>  7
<212>  PRT
<213>  Artificial Sequence

<220>
<223>  Palmitoyl hexapeptide-12


<220>
<221>  MISC_FEATURE
<222>  (1)..(1)
<223>  X= Palmitoyl

<400>  17

Xaa Val Gly Val Ala Pro Gly
1               5


<210>  18
<211>  5
<212>  PRT
<213>  Artificial Sequence

<220>
<223>  NY2

<400>  18

Lys Thr Thr Lys Ser
1               5


<210>  19
<211>  6
<212>  PRT
<213>  Artificial Sequence

<220>
<223>  GNY2

<400>  19

Gly Lys Thr Thr Lys Ser
1               5


<210>  20
<211>  6
<212>  PRT
<213>  Artificial Sequence

<220>
<223>  NY3a

<400>  20

Lys Thr Thr Lys Ser Asp
1               5


<210>  21

<211>  6
<212>  PRT
<213>  Artificial Sequence

<220>
<223>  NY3b

<400>  21

Lys Thr Thr Lys Ser Glu
1               5


<210>  22
<211>  6
<212>  PRT
<213>  Artificial Sequence

<220>
<223>  KTTKS derivative

<400>  22

Asp Lys Thr Thr Lys Ser
1               5


<210>  23
<211>  6
<212>  PRT
<213>  Artificial Sequence

<220>
<223>  KTTKS derivative

<400>  23

Glu Lys Thr Thr Lys Ser
1               5


<210>  24
<211>  7
<212>  PRT
<213>  Artificial Sequence

<220>
<223>  GKTTKS derivative

<400>  24

Gly Lys Thr Thr Lys Ser Asp
1               5


<210>  25
<211>  7
<212>  PRT
<213>  Artificial Sequence

<220>
<223>  GKTTKS derivative

<400>  25

Gly Lys Thr Thr Lys Ser Glu
1               5



<210>  26
<211>  30
<212>  PRT
<213>  Artificial Sequence

<220>
<223>  (NY3a)x5

<400>  26

Lys Thr Thr Lys Ser Asp Lys Thr Thr Lys Ser Asp Lys Thr Thr Lys
1               5                   10                  15


Ser Asp Lys Thr Thr Lys Ser Asp Lys Thr Thr Lys Ser Asp
            20                  25                  30



<210>  27
<211>  31
<212>  PRT
<213>  Artificial Sequence

<220>
<223>  (GNY3a)x5

<400>  27

Gly Lys Thr Thr Lys Ser Asp Lys Thr Thr Lys Ser Asp Lys Thr Thr
1               5                   10                  15


Lys Ser Asp Lys Thr Thr Lys Ser Asp Lys Thr Thr Lys Ser Asp
            20                  25                  30



<210>  28
<211>  30
<212>  PRT
<213>  Artificial Sequence

<220>
<223>  (NY3b)x5

<400>  28

Lys Thr Thr Lys Ser Glu Lys Thr Thr Lys Ser Glu Lys Thr Thr Lys
1               5                   10                  15


Ser Glu Lys Thr Thr Lys Ser Glu Lys Thr Thr Lys Ser Glu
            20                  25                  30



<210>  29
<211>  31
<212>  PRT

<213>  Artificial Sequence

<220>
<223>  (GNY3b)x5

<400>  29

Gly Lys Thr Thr Lys Ser Glu Lys Thr Thr Lys Ser Glu Lys Thr Thr
1               5                   10                  15

Lys Ser Glu Lys Thr Thr Lys Ser Glu Lys Thr Thr Lys Ser Glu
            20                  25                  30

<210>  30
<211>  5
<212>  PRT
<213>  Artificial Sequence

<220>
<223>  Elastin pentapeptide

<400>  30

Lys Gly Gly Val Gly
1               5

<210>  31
<211>  5
<212>  PRT
<213>  Artificial Sequence

<220>
<223>  Elastin pentapeptide

<400>  31

Val Gly Gly Val Gly
1               5

<210>  32
<211>  5
<212>  PRT
<213>  Artificial Sequence

<220>
<223>  Elastin pentapeptide

<400>  32

Gly Val Gly Val Pro
1               5

<210>  33
<211>  5
<212>  PRT
<213>  Artificial Sequence

<220>

<223>  Elastin pentapeptide

<220>
<221>  MISC_FEATURE
<222>  (4)..(4)
<223>  X= V, I or K

<400>  33

Val Pro Gly Xaa Gly
1               5

<210>  34
<211>  6
<212>  PRT
<213>  Artificial Sequence

<220>
<223>  Elastin pentapeptide

<400>  34

Val Gly Val Ala Pro Gly
1               5

<210>  35
<211>  7
<212>  PRT
<213>  Artificial Sequence

<220>
<223>  Elastin heptapeptide

<400>  35

Leu Gly Ala Gly Gly Ala Gly
1               5

<210>  36
<211>  9
<212>  PRT
<213>  Artificial Sequence

<220>
<223>  Elastin nonapeptide

<400>  36

Leu Gly Ala Gly Gly Ala Gly Val Leu
1               5

<210>  37
<211>  7
<212>  PRT
<213>  Artificial Sequence

<220>
<223>  Elastin peptide derivative

<400>  37

Val Gly Val Ala Pro Gly Asp
1               5


<210>  38
<211>  7
<212>  PRT
<213>  Artificial Sequence

<220>
<223>  Elastin peptide derivative

<400>  38

Val Gly Val Ala Pro Gly Glu
1               5


<210>  39
<211>  24
<212>  PRT
<213>  Artificial Sequence

<220>
<223>  ELP4

<400>  39

Val Gly Val Ala Pro Gly Val Gly Val Ala Pro Gly Val Gly Val Ala
1               5                   10                  15


Pro Gly Val Gly Val Ala Pro Gly
            20


<210>  40
<211>  28
<212>  PRT
<213>  Artificial Sequence

<220>
<223>  ELPE4

<400>  40

Val Gly Val Ala Pro Gly Glu Val Gly Val Ala Pro Gly Glu Val Gly
1               5                   10                  15


Val Ala Pro Gly Glu Val Gly Val Ala Pro Gly Glu
            20                  25


<210>  41
<211>  1251
<212>  DNA
<213>  Artificial Sequence

<220>

<223>  Gtclp

<400>  41
atggcagatt ataaagacga tgatgataaa gattacaaag acgatgacga caaagactat      60

aaagatgatg acgataaaga aaacctttat tttcaaggta gtcgtcgtcg tcaaggtgct     120

gctgccgcag ccgctggtgc agcaggatta atcttacttg tgttactttc attagtatct     180

ttacgtgctc atgaacgcaa accaacatct ttagttgaag gttcacgcac tacaatgatg     240

ccaacatgga aagtaactgg tacattaaat ccagttgaag aaacagagaa agaagtagaa     300

gaagaaccat cttcacctcc accaccagca gtacaagcta caccagatgt acctcaagag     360

caagaagccg aaccaagtca gtcagaatgg gttccaccac ctaaaccttg ggaaccatta     420

gctgaaaaaa atattggtcg tttagaacgt actaatcgtg aagtagttcg tggtgatatg     480

gaaaatgcaa acatgataaa aaaattacgt gctgctatgc gtttattaaa gaaacaattt     540

gcagctaaaa ttgtgaattt agaacaagct tatgatcgtc gtttagctcg tgaaagtcgt     600

attttacacc aacgtattaa tacagtagct atgcaacctg gtccaacagg catgtcaggt     660

cctcaaggtt taccaggtcc tactggtcca gcaggccaaa atggttctcc aggctcacca     720

ggagcaatgg gtccacaagg accaatgggt ccaagaggtt atcgtggttt acgtggtcca     780

cctggtgatc aaggtcgtcc tggtgataca ggtagacctg gtgctcctgg tattccaggt     840

caaattggtc aacgtggacc tattggtcca atgggtgttc aaggccctga aggtccacgt     900

ggtaatcctg gattaccagg tccaccaggt gcaccaggtg ttccaggtat tggtattcaa     960

ggaccagcag gtcctcctgg tccaccagga cgctttcctg ctacagaaca ttgtagctat    1020

gttactggtg aatgtgtaat taacggcaat gacattttcc atgctatgcg tgaaactggc    1080

gatgtaagtt gtccaagaaa ctactatgtt aaaggtgttg attacatcaa atgcaataat    1140

ggtgctgaat accacaatac tttacagtta cgtttaacat gttgtttatt aggtttaaac    1200

gagaatcttt atttccaagg ttatccttat gatgttccag attatgctta a             1251


<210>  42
<211>  540
<212>  DNA
<213>  Artificial Sequence

<220>
<223>  Gtccld

<400>  42
ggatccggtc catgttgtgg accacctggt cctccaggtc caccaggacc acctggacca      60

acaggtatga gtggaccaca aggtttacca ggtcctactg gtcctgctgg tcaaaacggt     120

tcaccaggtt ctccaggtgc aatgggtcca caaggtccta tgggaccacg tggttatcgt     180

ggtttacgtg gtccaccagg tgatcaaggt cgtcctggtg atacaggtcg tccaggtgct     240

ccaggtattc caggtcagat tggtcaacgt ggtccaattg gtcctatggg tgttcaagga 300

ccagaaggtc caagaggtaa tccaggcctt ccaggaccac caggtgcacc aggtgtacct 360

ggtattggca ttcaaggtcc tgctggccca cctggtccac ctggtcgttt tggtcctcct 420

ggccctccag gcccaccagg tcctcctggt ccatgttgcg gctatatccc agaagctcca 480

cgtgatggtc aagcttatgt tcgcaaagat ggtgaatggg tgttattatc aacattctta 540

<210> 43
<211> 440
<212> DNA
<213> Artificial Sequence

<220>
<223> Gtcld

<400> 43
gatgatgatg acaaagatta caaagacgat gatgataaag gatccggacc aacaggtatg 60

agtggaccac aaggtttacc aggtcctact ggtcctgctg gtcaaaacgg ttcaccaggt 120

tctccaggtg caatgggtcc acaaggtcct atgggaccac gtggttatcg tggtttacgt 180

ggtccaccag gtgatcaagg tcgtcctggt gatacaggtc gtccaggtgc tccaggtatt 240

ccaggtcaga ttggtcaacg tggtccaatt ggtcctatgg gtgttcaagg accagaaggt 300

ccaagaggta atccaggcct tccaggacca ccaggtgcac caggtgtacc tggtattggc 360

attcaaggtc ctgctggccc acctggtcca cctggtcgtt tttaagttta aacgtcgacc 420

tgcagagcta gctactgcag 440

<210> 44
<211> 18
<212> DNA
<213> Artificial Sequence

<220>
<223> nucleic sequence encoding VGVAPG

<400> 44
gtaggtgtag ctcctggt 18

<210> 45
<211> 18
<212> DNA
<213> Artificial Sequence

<220>
<223> nucleic sequence encoding VGVAPG

<400> 45
gttggtgttg ctcctgga 18

<210> 46
<211> 18

<212>  DNA
<213>  Artificial Sequence

<220>
<223>  nucleic sequence encoding VGVAPG

<400>  46
gtaggtgttg ctccaggt                                                    18


<210>  47
<211>  18
<212>  DNA
<213>  Artificial Sequence

<220>
<223>  nucleic sequence encoding VGVAPG

<400>  47
gtgggtgtag ctcctggt                                                    18


<210>  48
<211>  21
<212>  DNA
<213>  Artificial Sequence

<220>
<223>  nucleic sequence encoding VGVAPGE

<400>  48
gtaggtgtag ctcctggtga a                                                21


<210>  49
<211>  21
<212>  DNA
<213>  Artificial Sequence

<220>
<223>  nucleic sequence encoding VGVAPGE

<400>  49
gttggtgttg ctcctggaga a                                                21


<210>  50
<211>  21
<212>  DNA
<213>  Artificial Sequence

<220>
<223>  nucleic sequence encoding VGVAPGE

<400>  50
gtaggtgttg ctccaggtga a                                                21


<210>  51
<211>  21
<212>  DNA
<213>  Artificial Sequence

<220>
<223>  nucleic sequence encoding VGVAPGE

<400>  51
gtgggtgtag ctcctggtga a                                                21


<210>  52
<211>  15
<212>  DNA
<213>  Artificial Sequence

<220>
<223>  nucleic sequence encoding KTTKS

<400>  52
aaaacaacta aatca                                                       15


<210>  53
<211>  18
<212>  DNA
<213>  Artificial Sequence

<220>
<223>  nucleic sequence encoding GKTTKS

<400>  53
ggtaaaacaa ctaaatca                                                    18


<210>  54
<211>  72
<212>  DNA
<213>  Artificial Sequence

<220>
<223>  nucleic sequence encoding ELP4

<400>  54
gtaggtgtag ctcctggtgt tggtgttgct cctggagtag gtgttgctcc aggtgtgggt      60

gtagctcctg gt                                                          72


<210>  55
<211>  84
<212>  DNA
<213>  Artificial Sequence

<220>
<223>  nucleic sequence encoding ELPE4

<400>  55
gtaggtgtag ctcctggtga agttggtgtt gctcctggag aagtaggtgt tgctccaggt      60

gaagtgggtg tagctcctgg tgaa                                             84


<210>  56
<211>  90
<212>  DNA
<213>  Artificial Sequence

<220>
<223>  nucleic sequence encoding (NY3a)x5

<400>  56
aagactacca aaagtgataa aacaactaaa agcgataaga caactaaatc tgataaaaca      60

actaaatcag acaaaacaac aaaatcagat                                        90


<210>  57
<211>  93
<212>  DNA
<213>  Artificial Sequence

<220>
<223>  nucleic sequence encoding G(NY3a)x5

<400>  57
ggtaagacta ccaaaagtga taaaacaact aaaagcgata agacaactaa atctgataaa      60

acaactaaat cagacaaaac aacaaaatca gat                                    93


<210>  58
<211>  90
<212>  DNA
<213>  Artificial Sequence

<220>
<223>  nucleic sequence encoding (NY3b)x5

<400>  58
aaaacaacta aatcagaaaa aactacaaaa agtgagaaga ctacaaaatc tgagaaaaca      60

accaagtcag aaaagacaac aaaatcagaa                                        90


<210>  59
<211>  93
<212>  DNA
<213>  Artificial Sequence

<220>
<223>  nucleic sequence encoding G(NY3b)x5

<400>  59
ggtaaaacaa ctaaatcaga aaaaactaca aaaagtgaga agactacaaa atctgagaaa      60

acaaccaagt cagaaaagac aacaaaatca gaa                                    93


<210>  60
<211>  8
<212>  PRT
<213>  Artificial Sequence

<220>
<223>  Flag Tag

<400>  60

Asp Tyr Lys Asp Asp Asp Asp Lys

1               5


<210>  61
<211>  24
<212>  PRT
<213>  Artificial Sequence

<220>
<223>  3X Flag Tag

<400>  61

Asp Tyr Lys Asp Asp Asp Asp Lys Asp Tyr Lys Asp Asp Asp Asp Lys
1               5                   10                  15


Asp Tyr Lys Asp Asp Asp Asp Lys
            20


<210>  62
<211>  9
<212>  PRT
<213>  Artificial Sequence

<220>
<223>  HA Tag

<400>  62

Tyr Pro Tyr Asp Val Pro Asp Tyr Ala
1               5


<210>  63
<211>  6
<212>  PRT
<213>  Artificial Sequence

<220>
<223>  His Tag

<400>  63

His His His His His His
1               5


<210>  64
<211>  14
<212>  PRT
<213>  Artificial Sequence

<220>
<223>  LG

<400>  64

Arg Ser Gly Gly Gly Gly Ser Gly Gly Gly Gly Ser Gly Ser
1               5                   10

<210>  65
<211>  18
<212>  PRT
<213>  Artificial Sequence

<220>
<223>  LGM

<400>  65

Arg Ser Gly Gly Gly Gly Ser Ser Gly Gly Gly Gly Gly Gly Ser Ser
1               5                   10                  15


Arg Ser




<210>  66
<211>  424
<212>  DNA
<213>  Artificial Sequence

<220>
<223>  PpsbD-5'UTR

<400>  66
atgaaattaa atggatattt ggtacattta attccacaaa aatgtccaat acttaaaata      60

caaaattaaa agtattagtt gtaaacttga ctaacatttt aaattttaaa tttttcccta     120

attatatatt ttacttgcaa aatttataaa aattttatgc atttttatat cataataata     180

aaacctttat tcatggttta taatataata attgtgatga ctatgcacaa agcagttcta     240

gtcccatata tataactata tataacccgt ttaaagattt atttaaaaat atgtgtgtaa     300

aaaatgctta tttttaattt tattttatat aagttataat attaaataca caatgattaa     360

aattaaataa taataaattt aacgtaacga tgagttgttt ttttattttg gagatacacg     420

cacc                                                                  424


<210>  67
<211>  1025
<212>  DNA
<213>  Artificial Sequence

<220>
<223>  Prrn-5'UTRatpA

<400>  67
tttttaatta agtaggaact cggtatatgc tcttttgggg tcttattagc tagtattagt      60

taactaacaa aagatcaata ttttagtttg ttttatatat tttattactt aagtagtaag     120

gatttgcatt tagcaatctt aaatacttaa gtaataatct ataaataaaa tatattttcg     180

ctttaaaact tataaaaatt atttgctcgt tataagccta aaaaaacgta ggatctctac     240

gagatattac attgtttttt tctttaattg gctttaatat tactttgtat atataaacca     300

aagtacttgt taatagttat taaattatat taactataca gtacaaagaa attttttgct 360

aaaaaaagta tgttaacatt aaaaattttt gtttatacag actagtcaga ctcggggggc 420

aggcaacaaa tttatttatt gtcccgtaag ggaaggggga aaacaattat tattttactg 480

cggagcagct tgttattgaa attttattaa aaaaaaaata aaaatttgac aaaaaaaaat 540

aaaaaagtta aattaaaaac actgggaatg ttctacatca taaaaatcaa aagggtttaa 600

aatcccgaca aaatttaaac tttaaagagt ggcgcctacc ttttttttaa tttgcatgat 660

tttaatgctt atgctatctt ttttatttag tccataaaac ctttaaagga ccttttctta 720

tgggatattt atattttcct aacaaagcaa tcggcgtcat aaactttagt tgcttacgac 780

gcctgtggac gtccccccct tccccttacg ggcaagtaaa cttagggatt ttaatgcaat 840

aaataaattt gtcctcttcg ggcaaatgaa ttttagtatt taaatatgac aagggtgaac 900

cattactttt gttaacaagt gatcttacca ctcactattt ttgttgaatt ttaaacttat 960

ttaaaattct cgagaaagat ttttaaaata aactttttta atcttttatt tattttttct 1020

ttttt 1025

<210> 68
<211> 400
<212> DNA
<213> Artificial Sequence

<220>
<223> 3'UTRatpA

<400> 68
tttttaatta agtaggaact cggtatatgc tcttttgggg tcttattagc tagtattagt 60

taactaacaa aagatcaata ttttagtttg ttttatatat tttattactt aagtagtaag 120

gatttgcatt tagcaatctt aaatacttaa gtaataatct ataaataaaa tatattttcg 180

ctttaaaact tataaaaatt atttgctcgt tataagccta aaaaaacgta ggatctctac 240

gagatattac attgtttttt tctttaattg gctttaatat tactttgtat atataaacca 300

aagtacttgt taatagttat taaattatat taactataca gtacaaagaa attttttgct 360

aaaaaaagta tgttaacatt aaaaattttt gtttatacag 400

<210> 69
<211> 435
<212> DNA
<213> Artificial Sequence

<220>
<223> 3'UTRrbcL

<400> 69
aagcttgtac tcaagctcgt aacgaaggtc gtgaccttgc tcgtgaaggt ggcgacgtaa 60

ttcgttcagc ttgtaaatgg tctccagaac ttgctgctgc atgtgaagtt tggaaagaaa 120

ttaaattcga atttgatact attgacaaac tttaattttt atttttcatg atgtttatgt 180

gaatagcata aacatcgttt ttatttttta tggtgtttag gttaaatacc taaacatcat 240

tttacatttt taaaattaag ttctaaagtt atcttttgtt taaatttgcc tgtgctttat 300

aaattacgat gtgccagaaa aataaaatct tagcttttta ttatagaatt tatctttatg 360

tattatattt tataagtaat aaaagaaata gtaacatact aaagcggatg taactcaatc 420

ggtagagtgc gatcc 435

<210> 70
<211> 140
<212> PRT
<213> Artificial Sequence

<220>
<223> HA-SP-3F-FX-APRO

<400> 70

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala
    130                 135                 140

<210> 71
<211> 423
<212> DNA

<213>  Artificial Sequence

<220>
<223>  ha-sp-3f-fX-apro

<400>  71
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaagg atccattgaa     240

ggtcgtagac cagacttttg cttagaacca ccatatacag gtccatgtaa agctcgtatc     300

attcgctatt tctacaatgc aaaagcagga ttatgtcaaa catttgtgta tggaggttgt     360

cgtgctaaac gtaacaactt taaatcagct gaagattgta tgcgtacttg tggtggtgct     420

taa                                                                   423


<210>  72
<211>  41
<212>  PRT
<213>  Artificial Sequence

<220>
<223>  SP

<400>  72

Asn Asn Asn Asp Leu Phe Gln Ala Ser Arg Arg Arg Phe Leu Ala Gln
1               5                   10                  15


Leu Gly Gly Leu Thr Val Ala Gly Met Leu Gly Pro Ser Leu Leu Thr
            20                  25                  30


Pro Arg Arg Ala Thr Ala Ala Gln Ala
        35                  40


<210>  73
<211>  123
<212>  DNA
<213>  Artificial Sequence

<220>
<223>  sp

<400>  73
aataataacg acttatttca agcatcacgt cgtcgtttct tagctcaatt aggaggttta      60

acagttgctg gtatgttagg tccatcttta cttactccac gtagagctac agcagctcaa     120

gct                                                                   123


<210>  74
<211>  88

<212>  PRT
<213>  Artificial Sequence

<220>
<223>  3F-FX-APRO

<400>  74

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Ile Glu Gly Arg Arg Pro
            20                  25                  30

Asp Phe Cys Leu Glu Pro Pro Tyr Thr Gly Pro Cys Lys Ala Arg Ile
        35                  40                  45

Ile Arg Tyr Phe Tyr Asn Ala Lys Ala Gly Leu Cys Gln Thr Phe Val
    50                  55                  60

Tyr Gly Gly Cys Arg Ala Lys Arg Asn Asn Phe Lys Ser Ala Glu Asp
65                  70                  75                  80

Cys Met Arg Thr Cys Gly Gly Ala
                85

<210>  75
<211>  63
<212>  DNA
<213>  Artificial Sequence

<220>
<223>  O5'AS_GibaproBE

<400>  75
gcagtagcta gctctgcagg tcgacgttta aacttaagca ccaccacaag tacgcataca      60

atc                                                                    63

<210>  76
<211>  72
<212>  DNA
<213>  Artificial Sequence

<220>
<223>  O3'AS_GibaproBE

<400>  76
gatgatgatg acaaagatta caaagacgat gatgataaag gatccattga aggtcgtaga      60

ccagactttt gc                                                          72

<210>  77
<211>  1282
<212>  DNA

<213>  Artificial Sequence

<220>
<223>  Expression cassette PpsbD-5'UTRpsbD-ha-sp-3f-fX-apro-3'UTRatpA

<400>  77
atgaaattaa atggatattt ggtacattta attccacaaa aatgtccaat acttaaaata      60

caaaattaaa agtattagtt gtaaacttga ctaacatttt aaattttaaa ttttttccta     120

attatatatt ttacttgcaa aatttataaa aattttatgc atttttatat cataataata     180

aaacctttat tcatggttta taatataata attgtgatga ctatgcacaa agcagttcta     240

gtcccatata tataactata tataacccgt ttaaagattt atttaaaaat atgtgtgtaa     300

aaaatgctta tttttaattt tattttatat aagttataat attaaataca caatgattaa     360

aattaaataa taataaattt aacgtaacga tgagttgttt ttttattttg gagatacacg     420

caccatggca tatccttatg atgtaccaga ttatgccaat aataacgact tatttcaagc     480

atcacgtcgt cgtttcttag ctcaattagg aggtttaaca gttgctggta tgttaggtcc     540

atctttactt actccacgta gagctacagc agctcaagct gattacaaag atgatgacga     600

taaagactat aaagatgatg atgacaaaga ttacaaagac gatgatgata aaggatccat     660

tgaaggtcgt agaccagact tttgcttaga accaccatat acaggtccat gtaaagctcg     720

tatcattcgc tatttctaca atgcaaaagc aggattatgt caaacatttg tgtatggagg     780

ttgtcgtgct aaacgtaaca actttaaatc agctgaagat tgtatgcgta cttgtggtgg     840

tgcttaagtt taaacgtcga cctgcagagc tagctactgc agtttttaat taagtaggaa     900

ctcggtatat gctcttttgg ggtcttatta gctagtatta gttaactaac aaaagatcaa     960

tattttagtt tgttttatat attttattac ttaagtagta aggatttgca tttagcaatc    1020

ttaaatactt aagtaataat ctataaataa aatatatttt cgctttaaaa cttataaaaa    1080

ttatttgctc gttataagcc taaaaaaacg taggatctct acgagatatt acattgtttt    1140

tttctttaat tggctttaat attactttgt atatataaac caaagtactt gttaatagtt    1200

attaaattat attaactata cagtacaaag aaattttttg ctaaaaaaag tatgttaaca    1260

ttaaaaattt ttgtttatac ag                                              1282

<210>  78
<211>  160
<212>  PRT
<213>  Artificial Sequence

<220>
<223>  HA-SP-APRO-LG-FX-NY2-3F

<400>  78

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Arg Pro Asp Phe Cys Leu Glu Pro Pro Tyr Thr Gly
    50                  55                  60

Pro Cys Lys Ala Arg Ile Ile Arg Tyr Phe Tyr Asn Ala Lys Ala Gly
65                  70                  75                  80

Leu Cys Gln Thr Phe Val Tyr Gly Gly Cys Arg Ala Lys Arg Asn Asn
                85                  90                  95

Phe Lys Ser Ala Glu Asp Cys Met Arg Thr Cys Gly Gly Ala Arg Ser
            100                 105                 110

Gly Gly Gly Gly Ser Gly Gly Gly Gly Ser Gly Ser Ile Glu Gly Arg
        115                 120                 125

Lys Thr Thr Lys Ser Gly Leu Asn Asp Tyr Lys Asp Asp Asp Asp Lys
    130                 135                 140

Asp Tyr Lys Asp Asp Asp Asp Lys Asp Tyr Lys Asp Asp Asp Asp Lys
145                 150                 155                 160

<210> 79
<211> 483
<212> DNA
<213> Artificial Sequence

<220>
<223> ha-sp-apro-1G-fX-ny2-3f

<400> 79
atggcatatc cttatgatgt accagattat gccaataata acgacttatt tcaagcatca 60

cgtcgtcgtt tcttagctca attaggaggt ttaacagttg ctggtatgtt aggtccatct 120

ttacttactc cacgtagagc tacagcagct caagctagac cagacttttg cttagaacca 180

ccatatacag gtccatgtaa agctcgtatc attcgctatt tctacaatgc aaaagcagga 240

ttatgtcaaa catttgtgta tggaggttgt cgtgctaaac gtaacaactt taaatcagct 300

gaagattgta tgcgtacttg tggtggtgct agatctggtg gaggcggttc aggcggtgga 360

ggttcaggat ccattgaagg tcgtaaaaca actaaatcag gtttaaacga ttacaaagat 420

gatgacgata aagactataa agatgatgat gacaaagatt acaaagacga tgatgataaa 480

taa                                                                 483

<210>  80
<211>  108
<212>  PRT
<213>  Artificial Sequence

<220>
<223>  APRO-LG-FX-NY2-3F

<400>  80

Arg Pro Asp Phe Cys Leu Glu Pro Pro Tyr Thr Gly Pro Cys Lys Ala
1               5                   10                  15

Arg Ile Ile Arg Tyr Phe Tyr Asn Ala Lys Ala Gly Leu Cys Gln Thr
            20                  25                  30

Phe Val Tyr Gly Gly Cys Arg Ala Lys Arg Asn Asn Phe Lys Ser Ala
        35                  40                  45

Glu Asp Cys Met Arg Thr Cys Gly Gly Ala Arg Ser Gly Gly Gly Gly
    50                  55                  60

Ser Gly Gly Gly Gly Ser Gly Ser Ile Glu Gly Arg Lys Thr Thr Lys
65                  70                  75                  80

Ser Gly Leu Asn Asp Tyr Lys Asp Asp Asp Asp Lys Asp Tyr Lys Asp
                85                  90                  95

Asp Asp Asp Lys Asp Tyr Lys Asp Asp Asp Asp Lys
            100                 105

<210>  81
<211>  28
<212>  DNA
<213>  Artificial Sequence

<220>
<223>  O5'ASTatpA

<400>  81
caatgtaata tctcgtagag atcctacg                                      28

<210>  82
<211>  35
<212>  DNA
<213>  Artificial Sequence

<220>
<223>  O5'ASTatpA2

<400>  82
cctacttaat taaaaactgc agtagctagc tctgc                              35

<210>  83
<211>  34
<212>  DNA
<213>  Artificial Sequence

<220>
<223>  O3'SUTRpsbD

<400>  83
cgatgagttg tttttttatt ttggagatac acgc                                    34


<210>  84
<211>  116
<212>  DNA
<213>  Artificial Sequence

<220>
<223>  O5'Gibs-ELP4

<400>  84
ctgcagtagc tagctctgca ggtcgacgtt taaacttaag cgtaatctgg aacatcatat       60

ggataaccag gagctacacc cacacctgga gcaacaccta ctccaggagc aacacc          116


<210>  85
<211>  110
<212>  DNA
<213>  Artificial Sequence

<220>
<223>  O3'Gibs-ELP4

<400>  85
gctgaagatt gtatgcgtac ttgtggtggt gctgattaca aagacgatga tgacaaagta       60

ggtgtagctc ctggtgttgg tgttgctcct ggagtaggtg ttgctccagg                 110


<210>  86
<211>  62
<212>  DNA
<213>  Artificial Sequence

<220>
<223>  O5'Gibs501BE

<400>  86
ctgcagtagc tagctctgca ggtcgacgtt taaacttaac caggagctac acccacacct       60

gg                                                                      62


<210>  87
<211>  70
<212>  DNA
<213>  Artificial Sequence

<220>
<223>  O3\222Gibs501BE

<400>  87
gatgacaaag attacaaaga cgatgatgat aaaggatcca ttgaaggtcg tagaccagac        60

ttttgcttag                                                               70


<210>  88
<211>  172
<212>  PRT
<213>  Artificial Sequence

<220>
<223>  HA-SP-3F-FX-APRO-F-ELP4

<400>  88

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Asp Tyr Lys Asp
    130                 135                 140

Asp Asp Asp Lys Val Gly Val Ala Pro Gly Val Gly Val Ala Pro Gly
145                 150                 155                 160

Val Gly Val Ala Pro Gly Val Gly Val Ala Pro Gly
                165                 170


<210>  89
<211>  207

<212>  DNA
<213>  Artificial Sequence

<220>
<223>  FPCR-FELP4-HA

<400>  89
agatgatgat gacaaagatt acaaagacga tgatgataaa ggatccgatt acaaagacga      60

tgatgacaaa gtaggtgtag ctcctggtgt tggtgttgct cctggagtag gtgttgctcc      120

aggtgtgggt gtagctcctg gttatccata tgatgttcca gattacgctt aagtttaaac      180

gtcgacctgc agagctagct actgcag                                          207


<210>  90
<211>  62
<212>  DNA
<213>  Artificial Sequence

<220>
<223>   O5'Gibs02BE

<400>  90
ctgcagtagc tagctctgca ggtcgacgtt taaacttaag cgtaatctgg aacatcatat      60

gg                                                                     62


<210>  91
<211>  75
<212>  DNA
<213>  Artificial Sequence

<220>
<223>  O3'Gibs02BE

<400>  91
agatgatgat gacaaagatt acaaagacga tgatgataaa ggatccgatt acaaagacga      60

tgatgacaaa gtagg                                                       75


<210>  92
<211>  67
<212>  PRT
<213>  Artificial Sequence

<220>
<223>  3F-1F-ELP4 = 3F-F-ELP4

<400>  92

Asp Tyr Lys Asp Asp Asp Asp Lys Asp Tyr Lys Asp Asp Asp Asp Lys
1               5                   10                  15


Asp Tyr Lys Asp Asp Asp Asp Lys Gly Ser Asp Tyr Lys Asp Asp Asp
            20                  25                  30


Asp Lys Val Gly Val Ala Pro Gly Val Gly Val Ala Pro Gly Val Gly

35                  40                  45

Val Ala Pro Gly Val Gly Val Ala Pro Gly Tyr Pro Tyr Asp Val Pro
    50                  55                  60

Asp Tyr Ala
65

<210>  93
<211>  7
<212>  PRT
<213>  Artificial Sequence

<220>
<223>  TEV

<400>  93

Glu Asn Leu Tyr Phe Gln Gly
1               5

<210>  94
<211>  5
<212>  PRT
<213>  Artificial Sequence

<220>
<223>  EK

<400>  94

Asp Asp Asp Asp Lys
1               5

<210>  95
<211>  234
<212>  DNA
<213>  Artificial Sequence

<220>
<223>  lgm-tv-elpe4

<400>  95
cagctgaaga ttgtatgcgt acttgtggtg gtgctagatc tggtggaggc ggttcaagtg      60

gtggaggagg tggcggatct tcaagatctg aaaatttata ttttcaaggt gtaggtgtag     120

ctcctggtga agttggtgtt gctcctggag aagtaggtgt tgctccaggt gaagtgggtg     180

tagctcctgg tgaataagtt taaaccaggt gacctgcaga gctagctact gcag           234

<210>  96
<211>  231
<212>  DNA
<213>  Artificial Sequence

<220>

<223>  lgm-ek-elpe4

<400>  96
cagctgaaga ttgtatgcgt acttgtggtg gtgctagatc tggtggaggc ggttcaagtg      60

gtggaggagg tggcggatct tcaagatctg acgatgacga caagggtgta ggtgtagctc     120

ctggtgaagt tggtgttgct cctggagaag taggtgttgc tccaggtgaa gtgggtgtag     180

ctcctggtga ataagtttaa accaggtgac ctgcagagct agctactgca g              231


<210>  97
<211>  1282
<212>  DNA
<213>  Artificial Sequence

<220>
<223>  expression cassette of example 3

<400>  97
atgaaattaa atggatattt ggtacattta attccacaaa aatgtccaat acttaaaata      60

caaaattaaa agtattagtt gtaaacttga ctaacatttt aaattttaaa ttttttccta     120

attatatatt ttacttgcaa aatttataaa aattttatgc atttttatat cataataata     180

aaacctttat tcatggttta taatataata attgtgatga ctatgcacaa agcagttcta     240

gtcccatata tataactata tataacccgt ttaaagattt atttaaaaat atgtgtgtaa     300

aaaatgctta tttttaattt tattttatat aagttataat attaaataca caatgattaa     360

aattaaataa taataaattt aacgtaacga tgagttgttt ttttattttg gagatacacg     420

caccatggca tatccttatg atgtaccaga ttatgccaat aataacgact tatttcaagc     480

atcacgtcgt cgtttcttag ctcaattagg aggtttaaca gttgctggta tgttaggtcc     540

atctttactt actccacgta gagctacagc agctcaagct gattacaaag atgatgacga     600

taaagactat aaagatgatg atgacaaaga ttacaaagac gatgatgata aaggatccat     660

tgaaggtcgt agaccagact tttgcttaga accaccatat acaggtccat gtaaagctcg     720

tatcattcgc tatttctaca atgcaaaagc aggattatgt caaacatttg tgtatggagg     780

ttgtcgtgct aaacgtaaca actttaaatc agctgaagat tgtatgcgta cttgtggtgg     840

tgcttaagtt taaacgtcga cctgcagagc tagctactgc agtttttaat taagtaggaa     900

ctcggtatat gctcttttgg ggtcttatta gctagtatta gttaactaac aaaagatcaa     960

tattttagtt tgttttatat attttattac ttaagtagta aggatttgca tttagcaatc    1020

ttaaatactt aagtaataat ctataaataa aatatatttt cgctttaaaa cttataaaaa    1080

ttatttgctc gttataagcc taaaaaaacg taggatctct acgagatatt acattgtttt    1140

tttctttaat tggctttaat attactttgt atatataaac caaagtactt gttaatagtt    1200

attaaattat attaactata cagtacaaag aaattttttg ctaaaaaaag tatgttaaca    1260

ttaaaaattt ttgtttatac ag                                              1282

<210>  98
<211>  361
<212>  DNA
<213>  Artificial Sequence

<220>
<223>  GNC-ALTNY2

<400>  98
gatgatgatg acaaagatta caaagacgat gatgataaag gatccattga aggtcgtaga      60

ccagactttt gcttagaacc accatataca ggtccatgta aagctcgtat cattcgctat     120

ttctacaatg caaaagcagg attatgtcaa acatttgtgt atggaggttg tcgtgctaaa     180

cgtaacaact ttaaatcagc tgaagattgt atgcgtactt gtggtggtgc tagatctggt     240

ggaggcggtt caagtggtgg aggaggtggc ggatcttcaa gatctgaaaa tttatatttt     300

caaggtaaaa caactaaatc ataagtttaa accaggtgac ctgcagagct agctactgca     360

g                                                                     361

<210>  99
<211>  355
<212>  DNA
<213>  Artificial Sequence

<220>
<223>  GNC-ALENY2

<400>  99
gatgatgatg acaaagatta caaagacgat gatgataaag gatccattga aggtcgtaga      60

ccagactttt gcttagaacc accatataca ggtccatgta aagctcgtat cattcgctat     120

ttctacaatg caaaagcagg attatgtcaa acatttgtgt atggaggttg tcgtgctaaa     180

cgtaacaact ttaaatcagc tgaagattgt atgcgtactt gtggtggtgc tagatctggt     240

ggaggcggtt caagtggtgg aggaggtggc ggatcttcaa gatctgacga tgacgacaag     300

aaaacaacta aatcataagt ttaaaccagg tgacctgcag agctagctac tgcag          355

<210>  100
<211>  193
<212>  PRT
<213>  Artificial Sequence

<220>
<223>  HA-SP-3F-FX-APRO-LGM-TV-ELPE4

<400>  100

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Glu Asn
145                 150                 155                 160

Leu Tyr Phe Gln Gly Val Gly Val Ala Pro Gly Glu Val Gly Val Ala
                165                 170                 175

Pro Gly Glu Val Gly Val Ala Pro Gly Glu Val Gly Val Ala Pro Gly
            180                 185                 190

Glu

<210> 101
<211> 191
<212> PRT
<213> Artificial Sequence

<220>
<223> HA-SP-3F-FX-APRO-LGM-EK-ELPE4

<400> 101

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr

20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Asp Asp
145                 150                 155                 160

Asp Asp Lys Val Gly Val Ala Pro Gly Glu Val Gly Val Ala Pro Gly
                165                 170                 175

Glu Val Gly Val Ala Pro Gly Glu Val Gly Val Ala Pro Gly Glu
            180                 185                 190

<210>  102
<211>  4
<212>  PRT
<213>  Artificial Sequence

<220>
<223>  FX

<400>  102

Ile Glu Gly Arg
1

<210>  103
<211>  168
<212>  PRT
<213>  Artificial Sequence

<220>

<223>  HA-SP-3F-FX-APRO-LGM-EK-NY2

<400>  103

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Asp Asp
145                 150                 155                 160

Asp Asp Lys Lys Thr Thr Lys Ser
                165

<210>  104
<211>  507
<212>  DNA
<213>  Artificial Sequence

<220>
<223>  ha-sp-3f-fX-apro-lgm-ek-ny2

<400>  104
atggcatatc cttatgatgt accagattat gccaataata acgacttatt tcaagcatca      60

cgtcgtcgtt tcttagctca attaggaggt ttaacagttg ctggtatgtt aggtccatct      120

ttacttactc cacgtagagc tacagcagct caagctgatt acaaagatga tgacgataaa      180

gactataaag atgatgatga caaagattac aaagacgatg atgataaagg atccattgaa 240

ggtcgtagac cagacttttg cttagaacca ccatatacag gtccatgtaa agctcgtatc 300

attcgctatt tctacaatgc aaaagcagga ttatgtcaaa catttgtgta tggaggttgt 360

cgtgctaaac gtaacaactt taaatcagct gaagattgta tgcgtacttg tggtggtgct 420

agatctggtg gaggcggttc aagtggtgga ggaggtggcg gatcttcaag atctgacgat 480

gacgacaaga aaacaactaa atcataa 507

<210> 105
<211> 170
<212> PRT
<213> Artificial Sequence

<220>
<223> HA-SP-3F-FX-APRO-LGM-TV-NY2

<400> 105

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Glu Asn
145                 150                 155                 160

Leu Tyr Phe Gln Gly Lys Thr Thr Lys Ser
                165                 170

<210>  106
<211>  513
<212>  DNA
<213>  Artificial Sequence

<220>
<223>  ha-sp-3f-fX-apro-lgm-tv-ny2

<400>  106
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaagg atccattgaa     240

ggtcgtagac cagacttttg cttagaacca ccatatacag gtccatgtaa agctcgtatc     300

attcgctatt tctacaatgc aaaagcagga ttatgtcaaa catttgtgta tggaggttgt     360

cgtgctaaac gtaacaactt taaatcagct gaagattgta tgcgtacttg tggtggtgct     420

agatctggtg gaggcggttc aagtggtgga ggaggtggcg gatcttcaag atctgaaaat     480

ttatattttc aaggtaaaac aactaaatca taa                                  513

<210>  107
<211>  194
<212>  PRT
<213>  Artificial Sequence

<220>
<223>  HA-SP-3F-FX-APRO-LGM-EK-(NY3a)x5

<400>  107

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys

85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Asp Asp
145                 150                 155                 160

Asp Asp Lys Gly Lys Thr Thr Lys Ser Asp Lys Thr Thr Lys Ser Asp
                165                 170                 175

Lys Thr Thr Lys Ser Asp Lys Thr Thr Lys Ser Asp Lys Thr Thr Lys
            180                 185                 190

Ser Asp

<210>  108
<211>  585
<212>  DNA
<213>  Artificial Sequence

<220>
<223>  ha-sp-3f-fX-apro-lgm-ek-(ny3a)x5

<400>  108
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaagg atccattgaa     240

ggtcgtagac cagacttttg cttagaacca ccatatacag gtccatgtaa agctcgtatc     300

attcgctatt tctacaatgc aaaagcagga ttatgtcaaa catttgtgta tggaggttgt     360

cgtgctaaac gtaacaactt taaatcagct gaagattgta tgcgtacttg tggtggtgct     420

agatctggtg gaggcggttc aagtggtgga ggaggtggcg gatcttcaag atctgacgat     480

gacgacaagg gtaagactac caaaagtgat aaaacaacta aaagcgataa gacaactaaa     540

tctgataaaa caactaaatc agacaaaaca acaaaatcag attaa                     585

<210>  109
<211>  195

<212>  PRT
<213>  Artificial Sequence

<220>
<223>  HA-SP-3F-FX-APRO-LGM-TV-(NY3a)x5

<400>  109

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Glu Asn
145                 150                 155                 160

Leu Tyr Phe Gln Gly Lys Thr Thr Lys Ser Asp Lys Thr Thr Lys Ser
                165                 170                 175

Asp Lys Thr Thr Lys Ser Asp Lys Thr Thr Lys Ser Asp Lys Thr Thr
            180                 185                 190

Lys Ser Asp
        195

<210>  110
<211>  588
<212>  DNA

<213>  Artificial Sequence

<220>
<223>  ha-sp-3f-fX-apro-lgm-tv-(ny3a)x5

<400>  110
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaagg atccattgaa     240

ggtcgtagac cagacttttg cttagaacca ccatatacag gtccatgtaa agctcgtatc     300

attcgctatt tctacaatgc aaaagcagga ttatgtcaaa catttgtgta tggaggttgt     360

cgtgctaaac gtaacaactt taaatcagct gaagattgta tgcgtacttg tggtggtgct     420

agatctggtg gaggcggttc aagtggtgga ggaggtggcg gatcttcaag atctgaaaat     480

ttatattttc aaggtaagac taccaaaagt gataaaacaa ctaaaagcga taagacaact     540

aaatctgata aaacaactaa atcagacaaa acaacaaaat cagattaa                  588

<210>  111
<211>  194
<212>  PRT
<213>  Artificial Sequence

<220>
<223>  HA-SP-3F-FX-APRO-LGM-EK-(NY3b)x5

<400>  111

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Asp Asp
145                 150                 155                 160

Asp Asp Lys Gly Lys Thr Thr Lys Ser Glu Lys Thr Thr Lys Ser Glu
                165                 170                 175

Lys Thr Thr Lys Ser Glu Lys Thr Thr Lys Ser Glu Lys Thr Thr Lys
            180                 185                 190

Ser Glu

<210>  112
<211>  585
<212>  DNA
<213>  Artificial Sequence

<220>
<223>  ha-sp-3f-fX-apro-lgm-ek-(ny3b)x5

<400>  112
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaagg atccattgaa     240

ggtcgtagac cagacttttg cttagaacca ccatatacag gtccatgtaa agctcgtatc     300

attcgctatt tctacaatgc aaaagcagga ttatgtcaaa catttgtgta tggaggttgt     360

cgtgctaaac gtaacaactt taaatcagct gaagattgta tgcgtacttg tggtggtgct     420

agatctggtg gaggcggttc aagtggtgga ggaggtggcg gatcttcaag atctgacgat     480

gacgacaagg gtaaaacaac taaatcagaa aaaactacaa aaagtgagaa gactacaaaa     540

tctgagaaaa caaccaagtc agaaaagaca acaaaatcag aataa                     585

<210>  113
<211>  195
<212>  PRT
<213>  Artificial Sequence

<220>
<223>  HA-SP-3F-FX-APRO-LGM-TV-(NY3b)x5

<400>  113

Met Ala Tyr Pro Tyr Asp Val Pro Asp Tyr Ala Asn Asn Asn Asp Leu
1               5                   10                  15

Phe Gln Ala Ser Arg Arg Arg Phe Leu Ala Gln Leu Gly Gly Leu Thr
            20                  25                  30

Val Ala Gly Met Leu Gly Pro Ser Leu Leu Thr Pro Arg Arg Ala Thr
        35                  40                  45

Ala Ala Gln Ala Asp Tyr Lys Asp Asp Asp Asp Lys Asp Tyr Lys Asp
    50                  55                  60

Asp Asp Asp Lys Asp Tyr Lys Asp Asp Asp Asp Lys Gly Ser Ile Glu
65                  70                  75                  80

Gly Arg Arg Pro Asp Phe Cys Leu Glu Pro Pro Tyr Thr Gly Pro Cys
                85                  90                  95

Lys Ala Arg Ile Ile Arg Tyr Phe Tyr Asn Ala Lys Ala Gly Leu Cys
            100                 105                 110

Gln Thr Phe Val Tyr Gly Gly Cys Arg Ala Lys Arg Asn Asn Phe Lys
        115                 120                 125

Ser Ala Glu Asp Cys Met Arg Thr Cys Gly Gly Ala Arg Ser Gly Gly
    130                 135                 140

Gly Gly Ser Ser Gly Gly Gly Gly Gly Gly Ser Ser Arg Ser Glu Asn
145                 150                 155                 160

Leu Tyr Phe Gln Gly Lys Thr Thr Lys Ser Glu Lys Thr Thr Lys Ser
                165                 170                 175

Glu Lys Thr Thr Lys Ser Glu Lys Thr Thr Lys Ser Glu Lys Thr Thr
            180                 185                 190

Lys Ser Glu
        195


<210>  114
<211>  588
<212>  DNA
<213>  Artificial Sequence

<220>
<223>  ha-sp-3f-fX-apro-lgm-tv-(ny3b)x5

<400>  114
atggcatatc cttatgatgt accagattat gccaataata acgacttatt tcaagcatca      60

cgtcgtcgtt tcttagctca attaggaggt ttaacagttg ctggtatgtt aggtccatct     120

ttacttactc cacgtagagc tacagcagct caagctgatt acaaagatga tgacgataaa     180

gactataaag atgatgatga caaagattac aaagacgatg atgataaagg atccattgaa     240

ggtcgtagac cagacttttg cttagaacca ccatatacag gtccatgtaa agctcgtatc     300

attcgctatt tctacaatgc aaaagcagga ttatgtcaaa catttgtgta tggaggttgt     360

cgtgctaaac gtaacaactt taaatcagct gaagattgta tgcgtacttg tggtggtgct     420

agatctggtg gaggcggttc aagtggtgga ggaggtggcg gatcttcaag atctgaaaat     480

ttatattttc aaggtaaaac aactaaatca gaaaaaacta caaaaagtga gaagactaca     540

aaatctgaga aaacaaccaa gtcagaaaag acaacaaaat cagaataa                  588

<210>  115
<211>  143
<212>  PRT
<213>  Artificial Sequence

<220>
<223>  3F-FX-APRO-LGM-TV-(NY3b)x5

<400>  115

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Ile Glu Gly Arg Arg Pro
            20                  25                  30

Asp Phe Cys Leu Glu Pro Pro Tyr Thr Gly Pro Cys Lys Ala Arg Ile
        35                  40                  45

Ile Arg Tyr Phe Tyr Asn Ala Lys Ala Gly Leu Cys Gln Thr Phe Val
    50                  55                  60

Tyr Gly Gly Cys Arg Ala Lys Arg Asn Asn Phe Lys Ser Ala Glu Asp
65                  70                  75                  80

Cys Met Arg Thr Cys Gly Gly Ala Arg Ser Gly Gly Gly Gly Ser Ser
                85                  90                  95

Gly Gly Gly Gly Gly Gly Ser Ser Arg Ser Glu Asn Leu Tyr Phe Gln
            100                 105                 110

Gly Lys Thr Thr Lys Ser Glu Lys Thr Thr Lys Ser Glu Lys Thr Thr
        115                 120                 125

Lys Ser Glu Lys Thr Thr Lys Ser Glu Lys Thr Thr Lys Ser Glu
    130                 135                 140

<210>  116
<211>  142
<212>  PRT
<213>  Artificial Sequence

<220>
<223>  3F-FX-APRO-LGM-EK-(NY3b)x5

<400>  116

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Ile Glu Gly Arg Arg Pro
            20                  25                  30

Asp Phe Cys Leu Glu Pro Pro Tyr Thr Gly Pro Cys Lys Ala Arg Ile
        35                  40                  45

Ile Arg Tyr Phe Tyr Asn Ala Lys Ala Gly Leu Cys Gln Thr Phe Val
    50                  55                  60

Tyr Gly Gly Cys Arg Ala Lys Arg Asn Asn Phe Lys Ser Ala Glu Asp
65                  70                  75                  80

Cys Met Arg Thr Cys Gly Gly Ala Arg Ser Gly Gly Gly Gly Ser Ser
                85                  90                  95

Gly Gly Gly Gly Gly Gly Ser Ser Arg Ser Asp Asp Asp Asp Lys Gly
            100                 105                 110

Lys Thr Thr Lys Ser Glu Lys Thr Thr Lys Ser Glu Lys Thr Thr Lys
        115                 120                 125

Ser Glu Lys Thr Thr Lys Ser Glu Lys Thr Thr Lys Ser Glu
    130                 135                 140

<210>  117
<211>  143
<212>  PRT
<213>  Artificial Sequence

<220>
<223>  3F-FX-APRO-LGM-TV-(NY3a)x5

<400>  117

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Ile Glu Gly Arg Arg Pro
                20                  25                  30

Asp Phe Cys Leu Glu Pro Pro Tyr Thr Gly Pro Cys Lys Ala Arg Ile
        35                  40                  45

Ile Arg Tyr Phe Tyr Asn Ala Lys Ala Gly Leu Cys Gln Thr Phe Val
    50                  55                  60

Tyr Gly Gly Cys Arg Ala Lys Arg Asn Asn Phe Lys Ser Ala Glu Asp
65                  70                  75                  80

Cys Met Arg Thr Cys Gly Gly Ala Arg Ser Gly Gly Gly Gly Ser Ser
                85                  90                  95

Gly Gly Gly Gly Gly Gly Ser Ser Arg Ser Glu Asn Leu Tyr Phe Gln
            100                 105                 110

Gly Lys Thr Thr Lys Ser Asp Lys Thr Thr Lys Ser Asp Lys Thr Thr
        115                 120                 125

Lys Ser Asp Lys Thr Thr Lys Ser Asp Lys Thr Thr Lys Ser Asp
    130                 135                 140

<210>  118
<211>  142
<212>  PRT
<213>  Artificial Sequence

<220>
<223>  3F-FX-APRO-LGM-EK-(NY3a)x5

<400>  118

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Ile Glu Gly Arg Arg Pro
                20                  25                  30

Asp Phe Cys Leu Glu Pro Pro Tyr Thr Gly Pro Cys Lys Ala Arg Ile
        35                  40                  45

Ile Arg Tyr Phe Tyr Asn Ala Lys Ala Gly Leu Cys Gln Thr Phe Val
    50                  55                  60

Tyr Gly Gly Cys Arg Ala Lys Arg Asn Asn Phe Lys Ser Ala Glu Asp
65                  70                  75                  80

Cys Met Arg Thr Cys Gly Gly Ala Arg Ser Gly Gly Gly Gly Ser Ser
                85                  90                  95

Gly Gly Gly Gly Gly Gly Ser Ser Arg Ser Asp Asp Asp Asp Lys Gly
            100                 105                 110

Lys Thr Thr Lys Ser Asp Lys Thr Thr Lys Ser Asp Lys Thr Thr Lys
        115                 120                 125

Ser Asp Lys Thr Thr Lys Ser Asp Lys Thr Thr Lys Ser Asp
    130                 135                 140

<210>  119
<211>  116
<212>  PRT
<213>  Artificial Sequence

<220>
<223>  3F-FX-APRO-LGM-EK-NY2

<400>  119

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Ile Glu Gly Arg Arg Pro
            20                  25                  30

Asp Phe Cys Leu Glu Pro Pro Tyr Thr Gly Pro Cys Lys Ala Arg Ile
        35                  40                  45

Ile Arg Tyr Phe Tyr Asn Ala Lys Ala Gly Leu Cys Gln Thr Phe Val
    50                  55                  60

Tyr Gly Gly Cys Arg Ala Lys Arg Asn Asn Phe Lys Ser Ala Glu Asp
65                  70                  75                  80

Cys Met Arg Thr Cys Gly Gly Ala Arg Ser Gly Gly Gly Gly Ser Ser
                85                  90                  95

Gly Gly Gly Gly Gly Gly Ser Ser Arg Ser Asp Asp Asp Asp Lys Lys
            100                 105                 110

Thr Thr Lys Ser
        115

<210>  120
<211>  118
<212>  PRT
<213>  Artificial Sequence

<220>
<223>  3F-FX-APRO-LGM-TV-NY2

<400>  120

Asp Tyr Lys Asp Asp Asp Asp Lys Asp Tyr Lys Asp Asp Asp Asp Lys
1               5                   10                  15

Asp Tyr Lys Asp Asp Asp Asp Lys Gly Ser Ile Glu Gly Arg Arg Pro
            20                  25                  30

Asp Phe Cys Leu Glu Pro Pro Tyr Thr Gly Pro Cys Lys Ala Arg Ile
        35                  40                  45

Ile Arg Tyr Phe Tyr Asn Ala Lys Ala Gly Leu Cys Gln Thr Phe Val
    50                  55                  60

Tyr Gly Gly Cys Arg Ala Lys Arg Asn Asn Phe Lys Ser Ala Glu Asp
65                  70                  75                  80

Cys Met Arg Thr Cys Gly Gly Ala Arg Ser Gly Gly Gly Gly Ser Ser
                85                  90                  95

Gly Gly Gly Gly Gly Gly Ser Ser Arg Ser Glu Asn Leu Tyr Phe Gln
            100                 105                 110

Gly Lys Thr Thr Lys Ser
        115

<210>  121
<211>  237
<212>  DNA
<213>  Artificial Sequence

<220>
<223>  GNC-LENY3a2

<400>  121
cagctgaaga ttgtatgcgt acttgtggtg gtgctagatc tggtggaggc ggttcaagtg      60

gtggaggagg tggcggatct tcaagatctg acgatgacga caagggtaag actaccaaaa     120

gtgataaaac aactaaaagc gataagacaa ctaaatctga taaaacaact aaatcagaca     180

aaacaacaaa atcagattaa gtttaaacca ggtgacctgc agagctagct actgcag       237

<210>  122
<211>  237
<212>  DNA
<213>  Artificial Sequence

<220>
<223>  GNC-LENY3b1

<400>  122
cagctgaaga ttgtatgcgt acttgtggtg gtgctagatc tggtggaggc ggttcaagtg      60

gtggaggagg tggcggatct tcaagatctg acgatgacga caagggtaaa acaactaaat     120

cagaaaaaac tacaaaaagt gagaagacta caaaatctga gaaaacaacc aagtcagaaa     180

agacaacaaa atcagaataa gtttaaacca ggtgacctgc agagctagct actgcag        237


<210>  123
<211>  240
<212>  DNA
<213>  Artificial Sequence

<220>
<223>  GNC-LTNY3a2

<400>  123
cagctgaaga ttgtatgcgt acttgtggtg gtgctagatc tggtggaggc ggttcaagtg      60

gtggaggagg tggcggatct tcaagatctg aaaatttata ttttcaaggt aagactacca     120

aaagtgataa aacaactaaa agcgataaga caactaaatc tgataaaaca actaaatcag     180

acaaaacaac aaaatcagat taagtttaaa ccaggtgacc tgcagagcta gctactgcag     240


<210>  124
<211>  240
<212>  DNA
<213>  Artificial Sequence

<220>
<223>  GNC-LTNY3b1

<400>  124
cagctgaaga ttgtatgcgt acttgtggtg gtgctagatc tggtggaggc ggttcaagtg      60

gtggaggagg tggcggatct tcaagatctg aaaatttata ttttcaaggt aaaacaacta     120

aatcagaaaa aactacaaaa agtgagaaga ctacaaaatc tgagaaaaca accaagtcag     180

aaaagacaac aaaatcagaa taagtttaaa ccaggtgacc tgcagagcta gctactgcag     240


<210>  125
<211>  359
<212>  DNA
<213>  Artificial Sequence

<220>
<223>  FPCR-AP-FELP4

<400>  125
gatgacaaag attacaaaga cgatgatgat aaaggatcca ttgaaggtcg tagaccagac      60

ttttgcttag aaccaccata tacaggtcca tgtaaagctc gtatcattcg ctatttctac     120

aatgcaaaag caggattatg tcaaacattt gtgtatggag gttgtcgtgc taaacgtaac     180

aactttaaat cagctgaaga ttgtatgcgt acttgtggtg gtgctgatta caaagacgat     240

gatgacaaag taggtgtagc tcctggtgtt ggtgttgctc ctggagtagg tgttgctcca 300

ggtgtgggtg tagctcctgg ttaagtttaa acgtcgacct gcagagctag ctactgcag 359